# EUROPEAN PATENT SPECIFICATION

(11) **EP 1 998 811 B1**
(45) Date of publication and mention of the grant of the patent: **17.10.2012**
(21) Application number: 07797158.8
(22) Date of filing: 09.03.2007
(51) Int. Cl.: A61K 48/00

(54) **IMMUNOTHERAPY FOR IMMUNE SUPPRESSED PATIENTS**
IMMUNTHERAPIE FÜR PATIENTEN MIT UNTERDRÜCKTEN IMMUNREAKTIONEN
IMMUNOTHERAPIE POUR PATIENTS IMMUNODEPRIMES

(30) Priority: 14.03.2006 US 374783
(43) Date of publication of application: 10.12.2008
(73) Proprietor: IRX Therapeutics, Inc., New York, NY 10019 (US)
(72) Inventor: HADDEN, John, W., Cold Spring Harbor, NY 11724 (US); NAYLOR, Paul, Farmingdale, NY 11735 (US)
(74) Representative: Austin, Hedley William
(86) International application number: PCT/US2007/063633
(87) International publication number: WO 2007/136910

(56) References cited:
- WO-A-2005/025494
- US-A1- 2002 146 397
- DUEÑAS-GONZALEZ A ET AL: "A pilot study of perilymphatic leukocyte cytokine mixture (IRX-2) as neoadjuvant treatment for early stage cervical carcinoma" INTERNATIONAL IMMUNOPHARMACOLOGY, ELSEVIER, AMSTERDAM, NL, vol. 2, no. 7, 1 June 2002 (2002-06-01), pages 1007-1016, XP002442178 ISSN: 1567-5769
- BRANDWEIN H: "Session V: Strategies for immunization - IRX-2: A natural cytokine stimulant for cancer vaccines" CANCER IMMUNOLOGY AND IMMUNOTHERAPY, SPRINGER-VERLAG, BERLIN, DE, vol. 52, no. SUPPL. 1, 1 March 2003 (2003-03-01), pages S17-S18,S30, XP002442179 ISSN: 0340-7004
- NAYLOR PAUL H ET AL: "T cell targeted immune enhancement yields effective T cell adjuvants." INTERNATIONAL IMMUNOPHARMACOLOGY, vol. 3, no. 8, August 2003 (2003-08), pages 1205-1215, XP002523104 ISSN: 1567-5769
- EGAN JAMES E ET AL: "IRX-2, a novel in vivo immunotherapeutic, induces maturation and activation of human dendritic cells in vitro" JOURNAL OF IMMUNOTHERAPY, RAVEN PRESS, NEW YORK, NY, US, vol. 30, no. 6, 1 September 2007 (2007-09-01), pages 624-633, XP009095051 ISSN: 1053-8550
- HADDEN J W ET AL: "IRX-2 and thymosin alpha1 (Zadaxin) increase T lymphocytes in T lymphocytopenic mice and humans" ANNALS OF THE NEW YORK ACADEMY OF SCIENCES, NEW YORK ACADEMY OF SCIENCES, NEW YORK, NY, US, vol. 1112, 1 September 2007 (2007-09-01), pages 245-255, XP009101818 ISSN: 0077-8923
- MENESES A. ET AL.: 'Histologic Findings in Patients with Head and Neck Squamous Cell Carcinoma Recieving Perilymphatic Natural Cytokine Mixture (IRX-2) Prior to Surgery' ARCH. PATHOL. LAB MED. vol. 122, May 1998, pages 447 - 454, XP008034795

## Description

The invention concerns treating cellular immunodeficiency characterised by T lymphocytopenia, one or more dendritic cell functional defects (e.g those associated with lymph node sinus histiocytosis) and/or one or more monocyte functional defects (e.g. those associated with a negative CMI (cell-mediated immunity) skin test).

Cellular immunodeficiency is a deficiency of immune response in which the body cannot effectively protect itself from harmful antigens. The immune system in this condition is effectively turned off. Such deficiency can be drug-induced, e.g. by drug treatment, virus-induced, e.g. in AIDS, or induced by a disease state such as cancer. Cellular immunodeficiency is common among cancer patients; the body cannot protect against tumour antigens, allowing a tumour to grow and metastasize.

Cellular immunodeficiency, whether cancer related or not, can be due to T cell, dendritic cell and/or monocyte functional defects. For example, one or more T cell functional defects are believed to underlie T lymphocytopenia, a cellular immunodeficiency characterised by low blood T cell levels and impaired function of existing lymphocytes. To date, there is no generally accepted (clinically approved) way to treat T lymphocytopenia. Bone marrow transplants (with or without thymus transplants) have been used for severe combined immunodeficiency (SCID), whether the condition is congenital, irradiation- or chemotherapy-induced. Recombinant IL-2 (rIL-2) has been administered to AIDS patients with some effect, but with much toxicity. In general, the limited efficacy and significant toxicity associated with high doses of rIL-2, rIFN-γ, rTNF-α, and other monotherapies, suggests reconsideration of the use of natural combinations of cytokines in therapeutic strategies.

Ideally, to treat or overcome a cellular immunodeficiency such as T lymphocytopenia, augmentation of T cell development and proliferation, i.e., T cell regeneration, is desired. However, it is generally believed that new T cells cannot be generated in the adult human. For example, Mackall et al. in New England Journal of Medicine, Vol. 332, pp. 143-149 (1995) noted the inability of adults to generate new T cells, whereas children generally retain the ability to generate such cells. Since T lymphocytopenia is often seen in cancer patients, Mackall et al. discuss the problem of trying to replenish T cells following cancer chemotherapy and/or radiotherapy in adults. There is some evidence, however, that following bone marrow transplantation after intense chemotherapy, new T cells can be generated in adults.

Two approaches have been used in order to generate new T cells in an attempt to correct T lymphocytopenia, as for example, in cancer patients. One approach, rIL-2 therapy, seeks to expand T cells already in the periphery, that is, memory T cells that are CD45 RO+, for example, in the blood, lymph nodes, and spleen. The other approach involves enhancing the processing in the thymus of new T cells from bone marrow-derived precursors. This happens naturally in children but not in adults. These new cells are called recent "thymic émigrés" and have the surface marker of "naïve" T cells, i.e., CD45 RA. The term "naïve T cells" as herein defined relates to newly-produced T cells, even in adults, wherein these T cells have not yet been exposed to antigen. Such T cells are therefore not antigen-specific but are capable of becoming antigen-specific upon the presentation of antigen by a mature dendritic cell having antigen, such as tumor peptides, exposed thereon.

While T lymphocytopenia is believed to be due to T cell functional defects, other cellular immunodeficiencies can be traced to one or more monocyte or dendritic cell functional defects. Monocytes as defined herein are essentially synonymous with adherent peripheral blood mononuclear cells (PBMCs) and are precursors to myeloid-derived macrophages and dendritic cells.

Defects in monocyte function can have wide-ranging effects on immune function. For example, because monocytes/macrophages play an important role in the generation of cell-mediated immunity and inflammation, monocyte functional defects may correlate with negative or reduced cell-mediated immune responses such those detected by standard CMI or DTH tests. Correcting monocyte functional defects would therefore promote cell-mediated immune responses in patients, e.g., by enhancing Th1 cell proliferative and cytotoxic responses.

In addition, dendritic cells (DCs) are highly specialized antigen presenting cells (APCs) capable of establishing and controlling primary immune responses (Hart, 1997; Matzinger, 1994; Steinman, 1991). Immature DCs reside in peripheral tissues where they capture and process antigen for subsequent presentation within the context of MHC I/II molecules (Banchereau, 2000). Phenotypic and functional changes occur in DCs upon encounter with microbial, proinflammatory or T cell derived stimuli, a process referred to as maturation. Generally, mature DCs are associated with eliciting immunity compared to the tolerogenic properties of immature DC (Steinman, 2002). The functional characteristics of mature DCs as compared to immature DCs include reduced phagocytic/endocytic activity and subsequent increase in antigen presentation, a loss of CD1a antigen and gain of CD83 antigen expression, increased MHC II antigen expression, and increased expression of co-stimulatory and adhesion molecules such as CD86, CD40, and CD54 (Celia, 1996; Cella 1997; Schnurr, 2000; Berchtold, 1999). The cumulative effect of these changes results in the mature DC having the capacity to migrate to the T cell areas of the draining lymphoid organs where they encounter naive T cells and present antigen and co-stimulatory molecules to the T cells, which initiates an effective adaptive immune response (Randolph, 2001; Sozzani, 1998).

The impaired function of DCs in cancer-bearing hosts has been established for several types of cancers, including squamous cell head and neck cancer (hereinafter referred to as "H&NSCC"), lung, renal-cell, breast and colorectal cancer (Gabrilovich, 1997; Chaux, 1996; Almand, 2000; Nestle, 1997; Tas, 1993; Thurnher, 1996; Hoffmann, 2002). Characterized DC defects result in a failure to effectively and successfully present tumor antigens to T cells and such defects can be characterized in a variety of ways including down-regulation of components of the antigen-processing machinery, reduced expression of costimulatory molecules and a reduction in the number of DCs that infiltrate the tumor (Whiteside, 2004; Gabrilovich, 1997; Choux, 1997). Cancer patients also show a decrease in the absolute numbers of mature DCs in the peripheral blood and lymph nodes (Hoffmann, 2002; Almand, 2000). VEGF, a soluble factor commonly secreted by tumors, has been shown to increase the induction of apoptosis in DCs and negatively correlates with DC numbers in the tumor tissue and peripheral blood of patients with many different types of cancer, including H&NSCC (Lissoni, 2001; Saito, 1998; Smith, 2000). Overall, a lack of DC function negatively impacts current immunotherapeutic strategies and correlates with unsuccessful clinical outcomes. Correcting dendritic cell functional defects would increase the number of mature dendritic cells that can then interact with antigens, e.g., tumor antigens, to present such antigens to T cells for the activation of cell-mediated and antibody-mediated immunity in a patient.

For example, sinus histiocytosis (SH) is a lymph node pathology seen in cancer patients that is characterized by the accumulation in lymph nodes of large histiocytes containing immature dendritic cells which have ingested and processed tumor antigens but are unable to fully mature and present these tumor peptides to naive T cells. SH is believed to be caused by a defect in dendritic cell processing. Without the proper presentation of antigen to the T cells, these T cells are incapable of stimulating Th1 and Th2 effector cells, which stimulation normally leads to cell-mediated and antibody-mediated immunity, respectively, in the body.

A natural cytokine mixture, NCM (also referred to herein as IRX-2), has been previously shown by applicant in United States Patent No. 5,698,194 to be effective in promoting T cell development and function in aged, immunosuppressed mice. Specifically, NCM was shown to decrease the proportion of immature T cells and increase the proportion of mature T cells in the thymus. The NCM included IL-1, IL-2, IL-6, IL-8, IL-12, IFN-γ, TNF-α, GM-CSF, G-CSF, and IL-3, IL-4, IL-7 in trace-amounts.

It has also been recently shown by applicant that naive T cells can be generated in adult humans. One method to induce the production of naive T cells and expose the naive T cells to endogenous or exogenous antigens at an appropriate site can be accomplished by administrating an NCM along with low dose cyclophosphamide (CY), indomethacin (INDO), and zinc, as disclosed in United States Patent No. 6,977,072 to Hadden. Specifically, a method is disclosed for unblocking immunization at a regional lymph node of a patient through the administration of a NCM. The unblocking of immunization occurs by promoting differentiation and maturation of immature dendritic cells in a regional lymph node and thus allowing presentation by resulting mature dendritic cells of small peptides to T cells to promote the production ofT cells. The NCM administered includes IL-1, IL-2, IL-6, IL-8, IL-10, IL-12, IFN-γ, TNF-α, G-CSF, and GM-CSF.

Applicant's data in United States Patent No. 6,977,072 showed that the NCM of the invention plus low dose CY and INDO can increase the number of T cell precursors and T lymphocyte counts in patients with H&NSCC. The lymph nodes of patients with H&NSCC are often distinguished by T cell depletion and sinus histiocytosis. Over 90% of the patients responded to the treatment and a majority had greater than 50% tumor reduction. Preliminary data suggested that immunotherapy with the NCM in H&NSCC patients converts DCs in the lymph nodes from an immature CD86-/83⁺ phenotype into activated and mature CD86⁺/83⁺ DCs - (Hadden 2004). In addition, treatment of these lymphocytopenic cancer patients with the combination regimen of NCM, CY and INDO resulted in marked lymphocyte mobilization; where analyzed, these patients showed increases in CD45RA+ T cells (i.e., naive T cells). While this combination regimen has been shown to unblock immunization at a regional lymph node, there was not sufficient evidence from the data to indicate whether the NCM alone, i.e., without the accompanying treatment with CY and INDO, was capable of treating a cellular immunodeficiency characterized by T lymphocytopenia and/or the dendritic cell functional defects associated with lymph node sinus histiocytosis.

In addition, in US Patent Application No. 10/637869 (which was published as US Patent 7182942 and WO2005/025494), applicant provided data indicating that cancer patients having a negative intradermal skin test reaction to the NCM have a poor clinical prognosis. However, a certain number of patients were converted from a negative skin test response to a positive one upon treatment with NCM and these converted patients showed improved clinical and pathological responses. It was suggested that a negative skin test to NCM reflects a monocyte defect in the patient, whereby cell-mediated immune responses were deficient, and treatment with NCM can remedy this functional defect.

The present invention therefore provides, for use in the treatment of cellular immunodeficiency characterized by T lymphocytopenia, by one or more dendritic cell defects (such as those associated with lymph node sinus histiocytosis), and/or by one or more monocyte functional defects (such as those associated with a negative NCM skin test), a composition comprising a pharmaceutically acceptable carrier and a natural cytokine mixture as defined below, without accompanying administration of cyclophosphamide or indomethacin.

The natural cytokine mixture is prepared in the continuous presence of a 4-aminoquinolone antibiotic and with the continuous or pulsed presence of a mitogen, the cytokine mixture containing as active components the cytokines IL-1, IL-2, IL-6, IL-8, IFN-γ, and TNF-α; the IL-1 is at a concentration of 60 to 6,000 pcg per ml, the IL-2 is at a concentration of 600 to 60,000 pcg per ml, the IL-6 is at a concentration of 60 to 6,000 pcg per ml, the IL-8 is at a concentration of 6,000 to 600,000 pcg per ml and the IFN-γ and TNF-α are at a concentration of 200 to 20,000 pcg per ml. The cytokine mixture may additionally comprises IL-12, GM-CSF, and G-CSF.

The composition used according to the invention causes increased generation of naive T cells, increased activation and maturation of dendritic cells and increased activation and maturation of monocytes/macrophages.

Immunologic tests in patients with cancer have had limited usefulness in predicting treatment outcome. Many types of immunologic studies have helped to delineate immunologic defects in patients with cancer on an experimental basis but few tests have been feasibly applied clinically to diagnose and monitor these patients. Two tests have proved useful, namely: 1) lymphocyte counts, specifically T cells and subsets; and 2) skin reactivity to dinitrochlorobenzene (DNCB) as a test of cell-mediated immunity (CMI, also called delayed type hypersensitivity (DTH)). The latter test is cumbersome and requires immunization and challenge days later and is no longer used clinically. The former is used but not emphasized as a predictor of outcome. Thus, there is a great need for tests which will reflect the cancer patient's cellular immune status.

There are two different limbs of the immune system that elicit a DTH or CMI skin test response: the afferent (input) limb and the efferent (output) limb. The afferent limb involves antigen or mitogen-triggered T cell proliferation and cytokine production. The efferent limb involves cytokine-induced monocyte influx, and monokine production leading to inflammation measured by erythema and induration.

During the 1970's, several groups used a skin test with the T cell mitogen, phytohemagglutin (PHA). The PHA skin test appeared to give the same type of information as the DNCB skin test, i.e., responsive patients did well clinically and unresponsive patients did poorly. However, PHA stimulates both limbs of the response and therefore a negative PHA skin test can reflect several defects: insufficient T cells, depressed function of T cells, or a defect in monocyte function. Other T cell mitogens that can be used for predictive skin tests include anti-CD3 monoclonal antibodies, e.g., OKT3 (OrthoClone®).

The invention provides a treatment following a skin test that reflects the efferent limb response, i.e., the monocyte-dependent component of the immune response. The NCM composition can be use as a diagnostic skin test for predicting treatment outcome, e.g., in cancer patients.

In addition to the cellular immunodeficiencies noted above, in some cases, T cells are suppressed endogenously by cancer lesions. It would be advantageous to block this suppression so that the T cells can function normally to assist the immune system. In this regard, it has been noted that the anti-neoplastic agent, cyclophosphamide (CY), while immunosuppressive in high doses, acts to inhibit T suppressor cells, and thus enhances immune responses, when given in low doses (Berd et al.; Ehrke MJ, 2003). As such, CY is acting as a chemical inhibitor of immune suppression. While CY has been employed in effective immunotherapy of cancer patients (Weber J., 2000; Murphy 1999; Hadden, 1994), no data has yet shown an acceptable clinical response combined with low or no toxicity. Furthermore, because prostaglandins are known to be immunosuppressive, a compound that blocks the synthesis of prostaglandins, such as a non-steroidal anti-inflammatory agent, would be thought to be useful in inhibiting this immune suppression.

The use according to the invention may be treatment of cancer, in which patients suffer from cellular immunodeficiencies, either due to cancer therapies or due to the immunosuppressive effect of cancer itself. The use may also be treatment of other disease states characterized by cellular immunodeficiencies such as T lymphocytopenia or other secondary immunodeficiencies, such as those characterized by one or more monocyte or dendritic cell functional defects.

Reference will now be made to the accompanying drawings wherein:
Figure 1 is a bar graph showing lymph node size in normal controls, cancer controls or NCM-treated populations with H&NSCC;
Figure 2A is a bar graph showing T cell area;
Figure 2B shows density in normal controls, H&NSCC controls and H&NSCC patients treated with NCM;
Figure 3A is a bar graph comparing B cell area and Figure 3B is a bar graph comparing follicles in the three treatment groups;
Figure 4A shows a comparison of other cells and Figure 4B shows a comparison of sinus histiocytosis in the three treatment groups;
Figure 5 is a graph showing a Node B&T (B cell and T cell) and Tumor B&T fit plot;
Figure 6A is a bar graph illustrating the accumulation of partially immature CD83+ DCs in the lymph nodes of SH+ cancer patients;
Figure 6B is a bar graph showing an increase in the number of CD86+ activated DCs upon treatment with NCM (IRX-2);
Figure 7 is a graph showing that NCM (IRX-2) induces DC maturation as detected by increased CD83 expression on DCs;
Figure 8 depicts the effect of NCM on the morphology of monocyte-derived DCs in cytospin preparations. The cells treated with NCM (Figure 8B) exhibited the morphological characteristics of mature DCs such as cellular projections and large irregular shaped nuclei;
Figure 9 contains histograms showing down-regulation of CD1a antigen and up-regulation of MHCII, CD86, CD40, and CD54 (ICAM-1) antigen expression by PBMCs incubated with NCM (IRX-2). These changes indicate that NCM stimulates the maturation of DCs.
Figure 10 is a graph showing that NCM (IRX-2) reduces the endocytic activity of immature DCs, which reduced activity is indicative of DC maturation.
Figure 11 is a graph showing that NCM (IRX-2) enhances the T cell stimulatory capacity of DCs, which enhancement is indicative of DC maturation and activation;
Figure 12A is a bar graph showing that NCM (IRX-2) increases the number of DCs producing IL-12 intracellularly. IL-12 is a cytokine produced by mature activated DCs;
Figure 12B is a bar graph showing that NCM (IRX-2) increases the total amount of bioactive IL-12 secreted by DCs;
Figure 13 is a bar graph showing that NCM (IRX-2) decreases VEGF-mediated apoptosis in DCs, indicating a protective effect of NCM on DC survival;
Figure 14A contains two bar graphs depicting the increase in percentage of monocyteslmacrophages staining positive for the combination of activation markers, CD86, HLA-DR, CD80 and CD40, after treatment of adherent PBMCs with NCM, as determined by flow cytometry.
Figure 14B is a series of bar graphs depicting the increase in mean fluorescence intensity (MFI) for the activation markers, CD86, HLA-DR, CD80 and CD40, after treatment of adherent PBMCs with NCM, as determined by flow cytometry.
Figure 15 contains bar graphs demonstrating that the NCM of the invention activates monocytes/macrophages, i.e., induces the expression of activation markers, CD86, HLA-DR, CD80 and CD40, to a greater degree than TNF-α.
Figure 16 contains bar graphs demonstrating that the NCM of the invention activates monocyteslmacrophages, i.e., induces the activation markers, HLA-DR, CD86 and CD40, even in the presence of the immunosuppressing cytokine IL-10. The NCM is better at activating monocytes/macrophages than LPS, both in the presence and absence of IL-10.
Figure 17 is a bar graph demonstrating that the NCM of the invention stimulates the production of TNF-α from activated monocytes/macrophages and overcomes the immunosuppressive effects of IL-10. The NCM stimulated the production of TNF-α to a greater extent than LPS.

As used herein, the term "chemical inhibitor" denotes a chemotherapeutic agent that is not immunosuppressive (preferably used at low doses) and that has immunomodulatory effects so as to increase immunity and/or an immune response, e.g., by inhibiting immune suppression/suppressor mechanisms.

As used herein, the term "adjutant" denotes a composition with the ability to enhance the immune response to a particular antigen. Such agility is manifested by a significant increase in immune-mediated protection. To be effective, an adjuvant must be delivered at or near the site of antigen. Enhancement of immunity is typically manifested by a significant increase (usually greater than 10 fold) in the titer of antibody raised to the antigen. Enhancement of cellular immunity can be measured by a positive skin test, cytotoxic T cell assay, ELISPOT assay for IFN-γ or IL-2, or T cell infiltration into the tumor (as described below).

As used herein, the term "tumor associated antigen" denotes a protein or peptide or other molecule capable of inducing an immune response to a tumor. This can include, but is not limited to, PSMA peptides, MAGE peptides (Sahin, 1997; Wang, 1999), Papilloma virus peptides (E6 and E7), MAGE fragments, NY ESO-1 or other similar antigens. Previously, these antigens were not considered to be effective in treating patients based either on their size, i.e., they were considered too small, or they were previously thought to lack immunogenic properties (i.e., they were considered to be self antigens).

As used herein, "NCM" denotes a natural cytokine mixture, as defined and set forth in United States Patent Nos. 5,632,983 and 5,698,194. The NCM can include recombinant cytokines. Briefly, NCM Is prepared in the continuous presence of a 4-aminoquinolone antibiotic and with the continuous or pulsed presence of a mitogen, which in the preferred embodiment is PHA.

A goal of the invention is to promote the production of naive T cells. "Naïve" T cells are newly produced T cells, even in adults, which have not yet been exposed to antigen. Such T cells are non-specific yet capable of becoming specific upon presentation by a mature dendritic cell having antigen, such as tumour peptides, exposed thereon. In US Patent. 6,977,072, it was shown that the administration of a NCM, along with low dose cyclophosphamide and indomethacin, to immunosuppressed patients with head and neck cancer led to an increase in immature, naive T cells (bearing CD3 and CD45 RA antigens) in the blood of these patients (see, Example 1 below). This was one of the first demonstrations that adult humans can generate naive T cells. However, it was previously unknown whether a NCM *alone (i.e., without the accompanying administration of CY and INDO)* can produce the effects of the combination of NCM, CY, and INDO referred to above.

Thus, the present invention provides use of such an NCM without accompanying administration of non-steroidal anti-inflammatory drugs. As the data in Example 2 below shows, administration of NCM alone to cancer patients causes a significant increase in lymphocyte counts, and specifically, causes an increase in both CD3+ and CD4+ T cells. Thus, administration according to the invention of a NCM alone can achieve the desired effects previously obtained by the administration of a NCM in conjunction with low dose cyclophosphamide and indomethacin.

The NCM preferably contains a concentration of IL-1 from 150 -1,200 pcg/ml; a concentration of IL-2 from 3,000 to 12,000 pcg/ml; a concentration of IL-6 from 300 to 2,000 pcg/ml; a concentration of IL-8 from 20,000 to 180,000 pcg/ml; and concentrations of IFN-γ and TNF-α, respectively, from 1,000 to 4,000 pcg/ml.

Dendritic cells are highly specialized antigen presenting cells which can establish and control primary immune responses. They capture antigen, e.g., in the peripheral tissues, and migrate to the T cell areas of draining secondary lymphoid organs where they encounter naive T cells and present the antigen to the T cells, thus initiating an immune response to the antigen. One or more defects in dendritic cell function would have a deleterious effect on the immune system.

The use according to the invention may be for lymph node sinus histiocytosis, a lymph node pathology observed in cancer patients, believed to be associated with a dendritic cell functional defect, with an accumulation of partially immature CD83+, CD86- DCs. As noted, normal dendritic cells capture antigens at the site of infection or immunization and migrate to a downstream lymph node where the antigens are presented to naive T cells to promote immunity. The immature dendritic cells in the lymph nodes of patients with SH cannot effectively present antigens to naive T cells. Therefore, a goal of the present invention is to reverse sinus histiocytosis, i.e., by the promotion of dendritic cell maturation, e.g., in cancer patients having SH.

The data presented in Example 3 below shows that NCM consisting of the six critical cytokines of IL-1, IL-2. IL-6, IL-8, IFN-γ, and TNF-α induces DC maturation. For example, the NCM of the invention was shown to increase the expression of CD83, a key marker of DC maturation, on DCs. More specifically, the data contained in Example 3 demonstrates that the NCM of the invention is a potent activator of dendritic cells as measured by morphologic, phenotypic and functional criteria. Thus, NCM was shown to promote morphologic changes in DCs indicative of maturation. NCM also was shown to down-regulate CD1a antigen expression on the DC cell surface, to upregulate CD83 and MHC II antigen expression on the DC cell surface, and to increase the expression of T cell co-stimulatary and adhesion molecules, e.g., CD86, CD40, and CD54 (ICAM-1), on the DC cell surface. In addition, NCM was shown to down-regulate endocytic activity of DCs (which is consistent with maturation of the DCs), to enhance the T cell stimulatory activity of DCs (as demonstrated by increased MLR activity) and to increase the production of IL-12 from DCs, IL-12 itself being an essential factor in the differentiation of naïve CD4+ helper T cells (into Th1 cells) and the activation and proliferation of cellular and phagocytic components of the immune system. Finally, NCM was shown to reduce VEGF-induced apoptosis of DCs. This anti-apoptotic effect of NCM could play a crucial role in maintaining the survival of mature DCs within a tumor setting, allowing for prolonged antigen presentation and activation of tumor antigen-specific cytotoxic T lymphocytes.

Thus, the NCM can be used alone, i.e., without accompanying treatment with CY and INDO as previously suggested, to achieve the desired result of enhancing the production of mature DCs.

The use according to the invention may be for the treatment of a cellular immunodeficiency characterized by one or more monocyte functional defects, e.g., which lead to a negative skin test to NCM in a patient. Monocytes are precursors to both dendritic cells and macrophages and thus any monocyte functional defect can negatively affect various immunological processes in the body. In the past, a negative skin test to NCM has predicted a poor clinical response to treatment in cancer patients. As noted above, it is believed that such a negative skin test indicates one or more defects in monocyte function. Previously, a number of NCM skin test negative patients treated with NCM, CY, and INDO in combination were shown to be converted to a NCM skin test-positive condition, suggesting that this combination treatment corrected a monocyte functional defect. New data, as provided in Example 9 herein, demonstrates that NCM including the six critical cytokines, IL-1, IL-2, IL-6, 1L-8, IFN-γ, and TNF-α, alone, i.e., without accompanying treatment with CY and INDO, is responsible for correcting this monocyte functional defect. More specifically, the data herein demonstrates that NCM alone is a potent activator of monocytes/macrophages. For example, NCM signficantly increases activation markers of monocytes/macrophages. i.e., HTA-DR, CD86, CD40 and CD80. In addition, the NCM was shown to be a stronger activator of monocytes/macrophages than TNF-α or LPS and the NCM was able to continue activating the cells even in the presence of the immunosuppressing cytokine IL-10.

The invention is therefore useful in the treatment of cellular immunodeficiencies characterized by monocyte functional defects, such as those immunodeficiencies that are characterized by a negative skin test to NCM. The NCM administered to treat a monocyte functional defect according to the present invention preferably contains the six cytokines of IL-1, IL-2, IL-6, IL-8, IFN-γ, and TNF-α in the concentration ranges described above.

The NCM may be used in a skin test to predict treatment outcome. This skin test reflects only the efferent limb response, i.e., the monocyte-dependent component. US Patent Application 10/637,869 (published as WO2005/025494) and Example 5 below discuss using the NCM as a diagnostic skin test for predicting treatment outcome by administering an NCM intracutaneously and determining a response to the NCM within 24 hours. A negative skin test indicates unresponsiveness to the NCM and immunotherapy and predicts the failure of cancer patients to respond to surgery with or without radiotherapy. Examples 6 and 7 below demonstrate that the NCM skin test not only predicts response to NCM treatment and immunotherapy plus surgery ± radiotherapy, but also predicts overall survival, time to recurrence, and time to death.

Thus, the NCM can be used to predict treatment outcome in cancer patients, including response to surgery (with or without accompanying treatments such as radiotherapy or chemotherapy), overall survival, time to recurrence and time to death.

For any of the above embodiments, the following administration details and/or protocols for treatment are used:

Preferably, the NCM is injected around lymphatics that drain into lymph nodes regional to a lesion, such as a tumor or other persistent lesions being treated. Perilymphatic administration into the lymphatics which drain into the lymph nodes, regional to the lesion, such as a cancer, is critical. Peritumoral injection has been associated with little response, even progression and is thus contraindicated. A ten (10) day injection scheme is optimal and a twenty (20) day injection protocol, while effective clinically, tends to reduce the Th1 response and shift towards a less desirable Th2 response as measured by lymphoid infiltration into the cancer. Bilateral injections are effective. Where radical neck dissection has occurred, contralateral injection is effective.

The administration may be prior to or after surgery, radiotherapy, chemotherapy, or combinations thereof; during the recurrence of tumours, i.e., during a period where tumour growth is occurring again after a period where tumuors were thought to have disappeared or were in remission.

The NCM may be administered and dosed to promote optimal immunization either to exogenous or endogenous antigen, taking into account the clinical condition of the individual patient, the site and method of administration, scheduling of administration, patient age, sex, and body weight. The pharmaceutically "effective amount" for purposes herein is thus determined by such considerations as are known in the art. The amount must be effective to promote immunization, leading to, e.g., tumor reduction, tumor fragmentation and leukocyte infiltration, delayed recurrence or improved survival rate, or improvement or elimination of symptoms.

In the methods of the present invention, the compounds of the present invention can be administered in various ways. It should be noted that they can be administered as the compound or as a pharmaceutically acceptable derivative and can be administered alone or as an active ingredient in combination with pharmaceutically acceptable carriers, diluents, adjuvants and vehicles. The compounds can be administered intra- or subcutaneously, or peri- or intralymphatically, intranodally or intrasplenically or intramuscularly, intraperitoneally, and intrathorasicalty. Implants of the compounds can also be useful. The patient being-treated is a warm-blooded animal and, in particular, mammals including man. The pharmaceutically acceptable carriers, diluents, adjuvants and vehicles as well as implant carriers generally refer to inert, nontoxic solid or liquid fillers, diluents or encapsulating material not reacting with the active ingredients of the invention.

The doses can be single doses or multiple doses over a period of several days. When administering the compound of the present invention, it is generally formulated in a unit dosage injectable form (e.g., solution, suspension, or emulsion). The pharmaceutical formulations suitable for injection include sterile aqueous solutions or dispersions and sterile powders for reconstitution into sterile injectable solutions or dispersions. The carrier can be a solvent or dispersing medium containing, for example, water, ethanol, polyol (for example, glycerol, propylene glycol, liquid polyethylene glycol, and the like), suitable mixtures thereof, and vegetable oils.

Proper fluidity can be maintained, for example, by the use of a coating such as lecithin, by the maintenance of the required particle size in the case of dispersion and by the use of surfactants. Nonaqueous vehicles such a cottonseed oil, sesame oil, olive oil, soybean oil, corn oil, sunflower oil, or peanut oil and esters, such as isopropyl myristate, can also be used as solvent systems for compound compositions. Additionally, various additives which enhance the stability, sterility, and isotonicity of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. In many cases, it is desirable to include isotonic agents, for example, sugars, sodium chloride, and the like. Prolonged absorption of the injectable pharmaceutical form can be brought about by the use of agents delaying absorption, for example, aluminum monostearate and gelatin. According to the present invention, however, any vehicle, diluent, or additive used would have to be compatible with the compounds.

Sterile injectable solutions can be prepared by incorporating the compounds utilized in practicing the present invention in the required amount of the appropriate solvent with several of the other ingredients, as desired.

A pharmacological formulation used in the present invention can be administered to the patient in an injectable formulation containing any compatible carrier, such as various vehicles, additives, and diluents; or the compounds utilized in the present invention can be administered parenterally to the patient in the form of slow-release subcutaneous implants or targeted delivery systems such as monoclonal antibodies, vectored delivery, iontophoretic, polymer matrices, liposomes, and microspheres. Examples of delivery systems useful in the present invention include those disclosed in: U.S. Pat. Nos. 5,225,182; 5,169,383; 5,167,616; 4,959,217; 4,925,678; 4,487,603; 4,486,194; 4,447,233; 4,447,224; 4,439,196; and 4,475,196. Many other such implants, delivery systems, and modules are well known to those skilled in the art

### EXAMPLES

All steps relating to cell culture are performed under sterile conditions. General methods of cellular immunology not described herein are performed as described in general references for cellular immunology techniques such as Mishell and Shiigi (Selected Methods in Cellular Immunology, 1981) and are well known to those of skill in the art.

### Preparation of Natural Cytokine Mixture (NCM)

NCM (also referred to herein as IRX-2) is a defined mixture of cytokines produced under GMP conditions over a 24 hour period following stimulation of human peripheral blood mononuclear cells (PBMCs) by phytohemagglutinin (PHA) and ciprofoxacin. The source of the PBMCs is screened and tested buffy coats purchased from FDA licensed blood banks. After PHA stimulation, the mitogen is removed through centrifugation and washing. All cellular elements are removed by centrifugation, and DNA is removed by anion exchange chromatography. The cell free supernatant is filter sterilized and nanofiltered to permit viral removal and is designated IRX-2. Stringent QC testing that includes both bioassay and ELISA determination of cytokine levels assures the consistency of the IRX-2. Safety testing with respect to sterility, DNA, mycoplasma, endotoxin and virus testing for CMV and EBV are also part of the GMP process. IRX-2 has been given safely to over 150 patients in various clinical trials-and is currently in Phase I/II testing under an FDA approved IND.

More specifically, the NCM can be prepared as follows:

The buffy coat white cells of human blood from multiple HIV-negative hepatitis virus-negative donors are collected. In an alternative embodiment, animals could be the cell source for veterinary uses. The cells from the donors are pooled and layered on ficoll hypaque gradients (Pharmacia) to yield lymphocytes free of neutrophils and erythrocytes. Alternative methods could be used that would result in the same starting lymphocyte population as are known in the art.

The lymphocytes are washed and distributed in X-VIVO 10 media (Whittaker Bioproducts) in surface-activated cell culture flasks for selection of cell subsets. The flasks (MICROCELLECTOR™ T-25 Cell Culture Flasks) contain immobilized stimulants, i.e., mitogens, such as PHA. The immobilization process for the stimulants is as described by the manufacturer for immobilizing various substances for panning procedures, i.e., separating cells, in the flasks. Alternatively, the lymphocytes are exposed to stimulants, e.g., PHA, for 2-4 hours and then washed three times.

The cells are incubated for 24-48 hours in X VIVO-10 media with 80 µg/ml ciprofloxacin (Miles Lab) at 37°C in a CO₂/air incubator. Alternatively, RPMI 1640 media could be used (Webb et al. 1973). HSA (human serum albumin) may be added to stabilize further the interleukins if HSA-free media is used for generations. Generally, HSA is used at 0.1 to 0.5% (weight by volume). Following incubation the supernatants are poured off and collected. The supernatants are stored at 4° C to -70° C.

### Example 1

Local perilymphatic injections in the neck with NCM in addition to treatment with low dose CY (at 300 mg/m²), INDO (25 mg orally three times daily), and zinc (65 mg elemental zinc as the sulfate orally once a day) have induced clinical regressions in a high percentage of patients with squamous cell head and neck cancer (H&NSCC) (Hadden, 1994; Meneses, 199B; Barrera, 2000; Hadden, 2003; Menesis, 2003) with evidence of improved, recurrence-free survival. Overall, including minor responses (25%-50%), tumor shrinkage and reduction of tumor in pathological specimens, over 90% responded and the majority had greater than 50% tumor reduction.

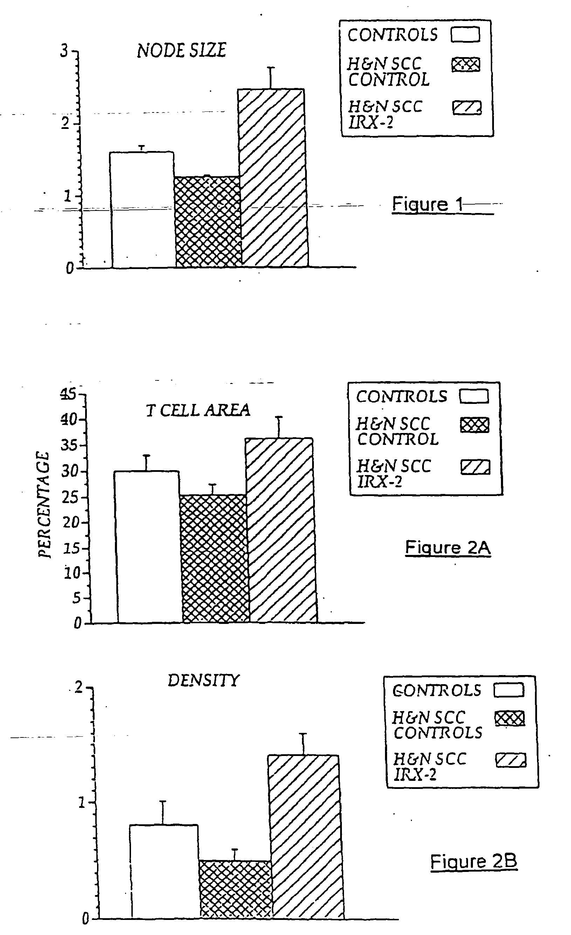

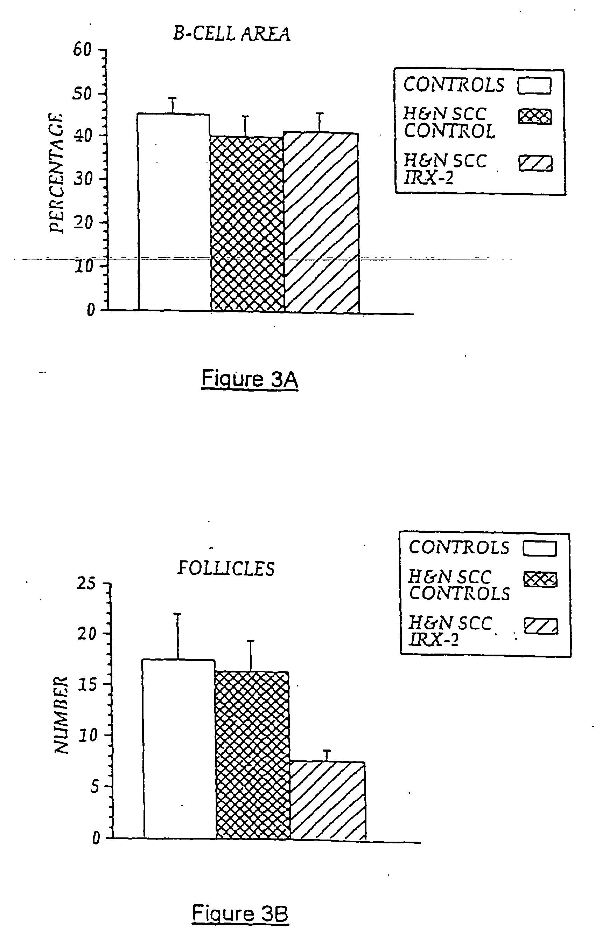

These responses are speculated to be mediated by immune regression since both B and T lymphocytes were observed infiltrating the tumors. The therapy was not associated with significant toxicity. Treatment of lymphocytopenic cancer patients with the combination of NCM has resulted in marked lymphocyte mobilization; where analyzed, these patients showed increases in CD45RA positive T cells (i.e., naive T cells (see Table I below)). Further, intratumoral or peritumoral injection of NCM in patients with H&NSCC resulted in either reversing immunotherapy-induced tumor regression or in progression of the tumor. The tumor is thus not the site of immunization. Rather, analysis of regional lymph nodes revealed that the regional lymph node is the site of immunization to postulated tumor antigens (Meneses, 2003; see Figures 1-5). None of these patients treated with NCM developed metastasis which would have been expected in 15% of the patients clinically and up to 50% pathologically. These results indicate systemic immunity rather than merely local immunity had been induced. Patients were pretested with a skin test to 0.1 ml of NCM prior to treatment and more than 90% of those with a positive skin test (>0.3mm at 24 hours) had robust clinical and pathological responses. Patients with negative skin tests had weak or no responses. Thus, skin testing selects good responders.

Major increases were observed in T lymphocyte counts (CD3) 752->1020 in these T lymphocytopoenic patients (T cell counts 752 vs. 1600 (normal)). Importantly, there was a corresponding -increase in "naïve" CD45RA positive T cells (532->782). As previously mentioned, these increases are generally not thought to occur in adults particularly with a pharmacological therapy like NCM. These cells presumably are recent thymic émigrés and could be considered a major new capacity for responding to new antigens like tumor antigens. The preexisting CD45RA positive cells were not responding to the tumor antigens and may have been incapable of doing so due to tumor induced immune suppression (anergy).

**Table I: Treatment of Lymphocytopoenic Patients with H&NSCC with NCM Increases in Naïve T Cells in Blood (#/mm)**

| | NAÏVE T CELL MARKER | | | PAN T CELL MARKER | | |
|---|---|---|---|---|---|---|
| PATIENT # | PRE | POST | INCREASE | PRE | POST | INCREASE |
| 1 | 479 | 778 | +299 | 704 | 1171 | +467 |
| 2 | 938 | 1309 | +371 | 1364 | 1249 | -115 |
| 3 | 98 | 139 | +41 | 146 | 178 | +32 |
| 4 | 341 | 438 | +97 | 655 | 590 | -65 |
| 5 | 567 | 652 | +97 | 453 | 643 | +190 |
| 6 | 658 | 1058 | +400 | 1118 | 1714 | +569 |
| 7 | 642 | 1101 | +459 | 822 | 1601 | +779 |
| MEAN | 532 | 782 | +250 | 752 | 1020 | +269 |

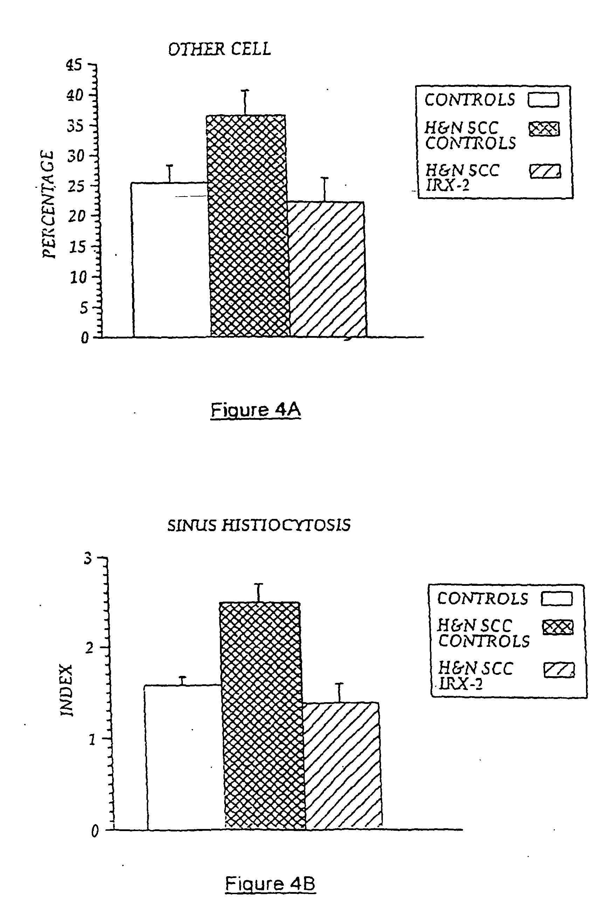

The literature (Hadden JW, Int'l J Immunopharmacol 11/12:629-644, 1997; Hadden JW, Int'l J Immunopharmacol 21:79-101, 1999) indicates that for both SCC and adenocarcinomas, the two major types of cancer, regional lymph nodes reflect abnormalities related to the tumor, including sinus histiocytosis, lymphoid depletion and often the presence of tumor-associated lymphocytes capable of reacting to tumor cells (with IL-2). With metastasis, lymphoid depletion and depressed function occur. An unpublished analysis of uninvolved cervical lymph nodes in 10 H&NSCC patients and 10 normal controls showed reduction in average lymph node size and an increase in sinus histiocytosis associated with H&NSCC (see Figures 1 -4A and B of the present application).

Following treatment with one cycle of the NCM protocol (Hadden, 1994; Meneses, 1998; Barrera, 2000), the uninvolved cervical lymph nodes showed the changes indicated in Figures 1-4. Compared to the regional lymph nodes of patients with H&NSCC not treated with NCM, these nodes showed a significant increase in size, T cell area and density, and decreases in number of germinal centers, sinus histiocytosis and congestion. The lymph nodes of treated patients were all stimulated and were larger than control nodes with increased T cell area and density. These nodes were thus not only restored to normal but showed evidence of T cell predominance, a known positive correlate with survival in H&NSCC (Hadden, 1997).

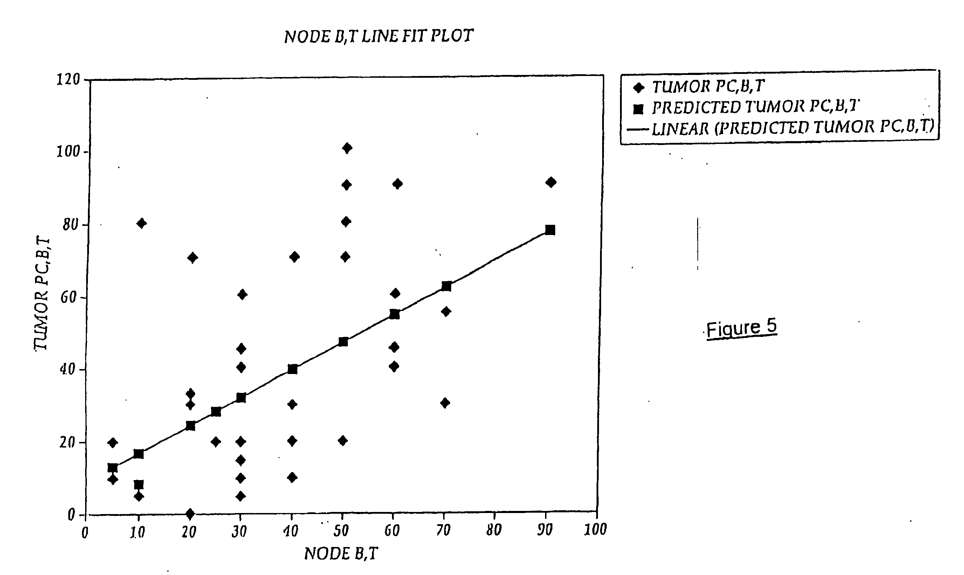

Importantly, when the lymph node changes related to B and T cell areas were correlated with the changes in their tumors reflecting T and B cell infiltration, a high degree of correlation was obtained for T cells (p.<0.01) and B cells (<0.01) and overall lymphoid presence (p.<0.001)(Figure 5). In turn, these changes correlated with tumor reduction by pathological and clinical criteria. These findings indicate that the tumor reactions are directly and positively correlated with lymph node changes and that the tumor reaction reflects the lymph node changes as the dependent variable. These findings, taken in conjunction with knowledge about how the immune system works in general (Roitt I, 1989), and following tumor transfection with a cytokine gene (Maass G, 1995), indicate that the NCM protocol immunizes these patients to yet unidentified tumor antigens at the level of the lymph nodes. No one has previously presented evidence for lymph node changes reflecting immunization with autologous tumor antigens. This confirms that the present invention can induce immunization with previously ineffective or poorly effective tumor antigens in an effect to yield regression of distant metastases.

### Example 2

### Correction by NCM of T lymphocytopenia

The objective of the following experiment was to assess the effect of a 10-daily injection treatment of NCM containing the six cytokines of IL-1, IL-2, IL-6, IL-8, IFN-γ, and TNF-α (115 units IL-2 equivalence/day) on lymphocyte counts (LC) of lymphocytopenic patients. These patients had recovered from prior surgery and radiotherapy for head and neck cancer, and had persistent lymphocytopenia with mean counts of 441 cells/mm³. Normal levels of LC are 2000 cells/mm³. The patients were free of cancer at the time of treatment. LC were obtained at day 0 and day 13. T lymphocytes (CD3+) and T cell subsets (CD4+ or CD8+) were assessed by cytofluorometry. Table II presents the data for five responding patients. Significant increases were observed for LC, CD3+, and CD4+ T cells.

**Table II**

| Pt. Number | TLC^{*} | CD3^{*} | CD4^{*} | CD8^{*} |
|---|---|---|---|---|
| 1 | 100 | 83 | 28 | 40 |
| 2 | 136 | 62 | 52 | 55 |
| 3 | 100 | 63 | 24 | 3 |
| 4 | 100 | 74 | 331 | -20 |
| 5 | 100 | 166 | 173 | -16 |
| Mean ± SEM | 107 ± 7 | 90 ± 19 | 122 ± 59 | 12 ± 15 |

| | | | | |
|---|---|---|---|---|
| *Changes in number of cells per mm³ from day 0 to day 13. | | | | |

These changes compare favorably to those achieved by much higher doses of pegylated interleukin 2 (3 x 10⁶ units of recombinant IL-2) in lymphocytopenic AIDS patients (T. Merigan, personal communication) but with less toxicity. They are less than those achieved with 8-day infusions of > 10 x 10⁶ units/day of IL-2 in AIDS patients; however, the latter required great expense, inconvenience, and had significant toxicity (Kovaks, et al. 1997). These results with NCM were obtained in the absence of INDO and CY and thus show that the effect of the regimen on LC is that of NCM and not an effect of INDO and CY.

### Example 3

### Correction by NCM of Dendritic Cell Defect(s) in Cancer:

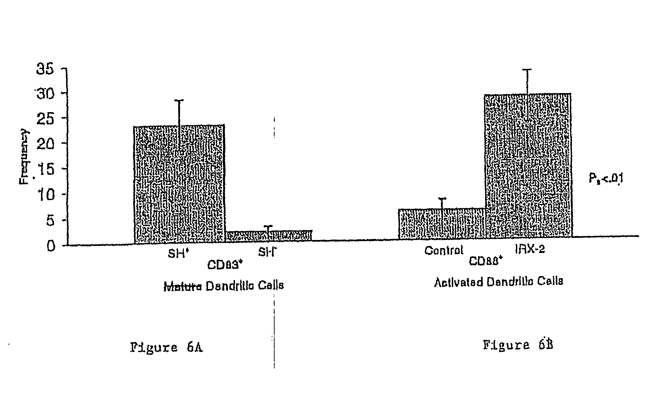

In previous experiments, lymph nodes from five NCM-treated H&NSCC patients and five untreated H&NSCC control patients were isolated and cellular constituents analyzed by flow cytometry using a panel of cell surface markers for dendritic cells (i.e., CD83+, CD86+, and CD68+). As noted above, sinus histiocytosis is a lymph node pathology seen in some cancer patients that is characterized by the accumulation in the lymph nodes of large histiocytes containing immature dendritic cells. As demonstrated in Figure 6A, patients with SH (SH+) have an accumulation of CD68+, CD83+, CD86-DCs in their lymph nodes, while those without noticeable SH have few CD83+ cells. However, NCM treatment resulted in a five-times increase in the number of CD86+ (concomitant with CD68+, CD83+) DCs compared to non-treated cancer controls, indicating a conversion to an "activated" DC phenotype. Controls are untreated H&NSCC patients compared to NCM-treated cancer patients (see Figure 6B).

Since sinus histiocytosis represents an accumulation of partially matured DCs presumed to be bearing endogenous tumor peptides, full maturation and activation with expression of the co-stimulatory receptor CD86 reflects use of the NCM of the present invention to correct this defect on maturation and to allow effective antigen presentation to T cells. The NCM of the present invention thus reverses sinus histiocytosis and leads to effective immunization of "naïve" T-cells.

The data described above and subsequent data contained in Meneses et al. (2003) showed that the treatment of patients with H&NSCC using perilymphatic NCM, low dose CY, and INDO reversed the sinus histiocytosis frequently evident in the lymph nodes in this and other cancers. However, it was not apparent from this data which of the above agents, NCM, CY, and/or INDO, corrected this defect.

The following data presents evidence that NCM containing the six cytokines of IL-1, IL-2, IL-6, IL-8, IFN-γ, and TNF-α induces DC maturation in the absence of CY and/or INDO. The NCM (IRX-2) used in these experiments contains several cytokines, preferably the six critical cytokines as discussed above or as shown in Table III below. For the purposes of these experiments, NCM (IRX-2) concentrations are expressed as the concentration of TNF-α contained in IRX-2. The cytokine concentration in IRX-2, including TNF-α, was measured by ELISA and the recombinant TNF-α purity is > 95%. For all experiments, except titrations, NCM was used at a concentration of 1 ng/ml.

**Table III: Cytokine levels in IRX-2 formulation Lot 041304 (ng/ml)**

| IL-1β | IL-2 | IFN-γ | TNF-α | IL-8 | IL-6 | IL-10 | G-CSF | GM-CSF |
|---|---|---|---|---|---|---|---|---|
| 0.3 | 4.2 | 2.2 | 1.0 | 25.2 | 0.7 | 0.03 | 0.06 | 0.4 |

The medium used was RPMI 1640, supplemented with 2 mM L-glutamine, 50 µg/ml streptomycin, 50 U/ml penicillin and 10% FBS (all reagents purchased from Cellgro, Herndon, VA). GM-CSF, TNF-α and VEGF₁₆₅ were purchased from Peprotech (Rocky Hill, NJ). X-VIVO 10 was purchased from BioWhittaker (Walkersville, MD). LPS was purchased from Sigma (St Louis, MO). All reagents were tested for endotoxin contamination with the sensitive *Limulus* amebocyte lysate assay (LAL assay; BioWhittaker) according to the manufacturer's instructions and were found to be negative. All solutions were found to contain less than 0.06 EU/ml, the lowest detection limit. Additionally, all plastic ware was pyrogen-free.

PBMCs used in these experiments were obtained from 30 ml of leukocyte enriched buffy coat of healthy donors by centrifugation with Ficoll-Hypaque centrifugation (Cellgro, Herndon, VA), and the light density fraction from the 42.5-50% interface was recovered. The cells were resuspended in culture medium and allowed to adhere to 6-well plates (Costar, Cambridge, MA). After 2 hours at 37° C, nonadherent cells were removed by washing and adherent cells (~90% CD14⁺ cells, i.e., monocytes) were cultured in 3 ml of medium supplemented with 50 ng/ml GM-CSF (500 U/ml) and 50 ng/ml IL-4 (500 U/ml).

For surface marker analysis, the following fluorochrome-conjugated mAbs (all from BD Pharmingen, San Diego, CA) were used: CD86-PE, CD80-FITC, CD54-APC, CD83-PE, HLA-DR-FITC, CD1a-APC, CD40-FITC and appropriate isotype controls. Immunophenotypic analysis was performed using FACS. Cells (0.25x10⁶) were washed in PBS supplemented with 2% FBS and 0.1% NaN₃ (FACS wash buffer) and incubated for 30 min at room temperature with APC-, PE-, or FITC-conjugated mAbs or with the corresponding isotype-matched mAb. Excess mAb was removed by washing in FACS wash buffer. Results were expressed as either mean fluorescence intensity or percentage of cells expressing the specified antigen. Fluorescence analysis was performed on a FACSCalibur flow cytometer (BD Biosciences, Rockville, MD) after acquisition of 10,000 events and analyzed with BD Biosciences CellQuest software (Rockville, MD).

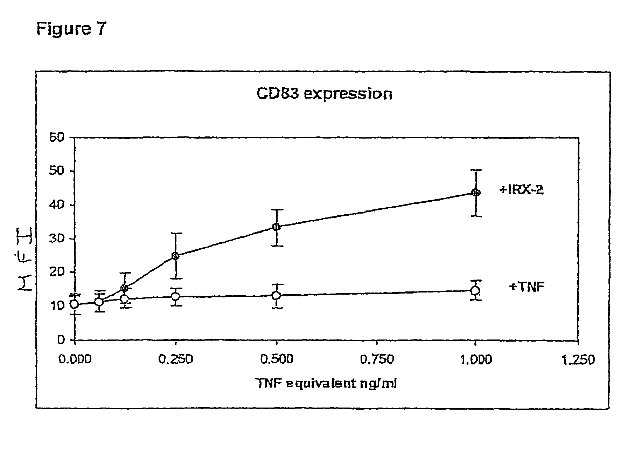

As demonstrated in Figure 7, NCM alone (without the presence of CY and/or INDO) increased the number of DCs bearing the CD83 antigen, a key marker of DC maturation. More specifically, adherent PBMCs (peripheral blood mononuclear cells) were cultured for 7 days in the presence of GM-CSF and IL-4 as described above and then stimulated with increasing amounts of either recombinant TNF-α (PeproTech) or NCM (IRX-2). After 48 hrs, the cells were washed and analyzed for CD83 expression by flow cytometry. Figure 7 indicates that NCM is active at inducing DC maturation, as evidenced by an increase in CD83+ cells. Moreover, NCM was more active at inducing DC maturation than an equivalent dose of TNF-α alone. The data in Figure 7 is represented as the mean of 5 individual experiments -/+ SEM (p<0.0001, by ANOVA).

This data indicates that NCM alone promotes the maturation of DCs and does so in a way that cannot be accounted for by any single cytokine contained in the NCM mixture that is known to act on DC maturation. For example, normal *in vitro* differentiation of PBMCs requires the presence of 100-500 U/ml GM-CSF (approximately 10-50 ng/ml) and 500-1000 U/ml IL-4 (50-100 ng/ml). This generates a population of cells committed to the DC lineage but in a relatively immature state (low/moderate CD86, CD40, HLA-DR expression, null for CD83). Undiluted NCM has undetectable quantities of IL-4 and contains 10 to 50-fold lower concentrations of GM-CSF (approximately 1.1 ng/ml) than is required for *in vitro* differentiation of DCs. Thus, the individual IL-4 and GM-CSF cytokines in the NCM cannot account for the CD83+ cells produced in the cultures of Figure 7.

TNF-α can induce such cells but at concentrations well above those contained in the NCM of the invention (see Figure 7). For example, after initial commitment to the dendritic cell lineage (by several days of GM-CSF + IL-4 *in vitro*), subsequent addition of a "danger signal" such as that derived from a pathogen (e.g., LPS) induces a fully mature dendritic cell phenotype including high/strong expression of CD86, CD40, HLA-DR, and the presence of CD83. TNF-α in the range of 20-50 ng/ml can largely mimic such a pathogen-derived danger signal resulting in upregulation of the same markers. However, the undiluted NCM mixture has only 2.8 ng/m) of TNF-α on average, far below the TNF-α concentrations required for full DC maturation. Thus, the results depicted in Figure 7 clearly demonstrate that, at the TNF-α equivalent concentrations used in this experiment, the induction of the CD83 marker by NCM could not be due to the presence of the TNF-α in the NCM mixture.

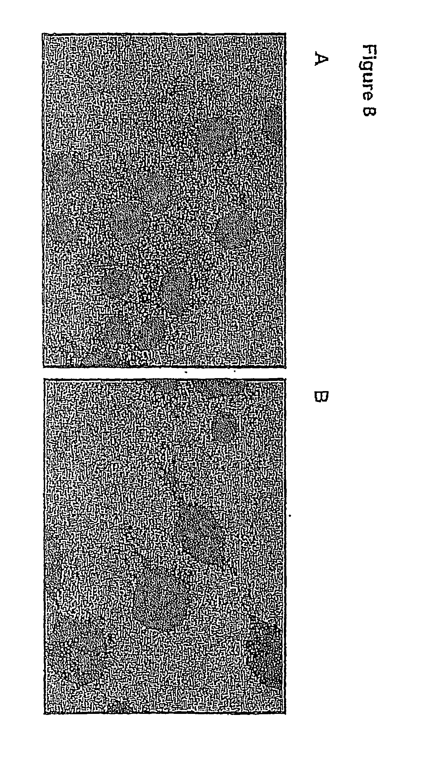

Since it is known that DCs undergo distinct morphological changes as they progress from immature to mature cells, immature DCs were treated with NCM to determine if NCM treatment changed the morphology of the cells. More specifically, adherent PBMCs were grown in the presence of GM-CSF (500U/ml) and IL-4 (500 U/ml) for 4 days as described above (which treatment is known to yield immature DCs) and then were either treated with NCM (IRX-2) or left untreated as controls. After 3 days, the cells were visualized by Wright staining and microscopy. As shown in Figure 8, the cells treated with NCM (Figure 8B) exhibited the characteristic cellular projections and motility of mature DCs, and continually extended and retracted their cellular processes and veils. These cells had large irregular shaped nuclei, numerous vesicles, relatively few cytoplasmic granules, and noticeable and abundant cellular projections as compared to the untreated controls (Figure 8A). Thus, NCM treatment resulted in DCs that possessed typical mature DC morphology.

In addition, it is known that the prototypical transition from immature to mature DCs results in well characterized increases and decreases in certain cell surface antigens. For example, immature DCs express high levels of CD1a, and upon encounter with stimuli such as cytokines or bacterial products, this marker is down-regulated. Thus, to determine if NCM treatment resulted in the gain or loss of cell surface markers associated with the activation and maturation of DCs, GM-CSF and IL-4-treated adherent PBMCs (monocytes) (as described above) were grown for 7 days and then incubated for 48 hrs with or without NMC (IRX-2) Expression of CD1a, HLA-DR, CD86, CD40 and CD54 was examined by flow cytometry and expressed as mean fluorescence intensity.

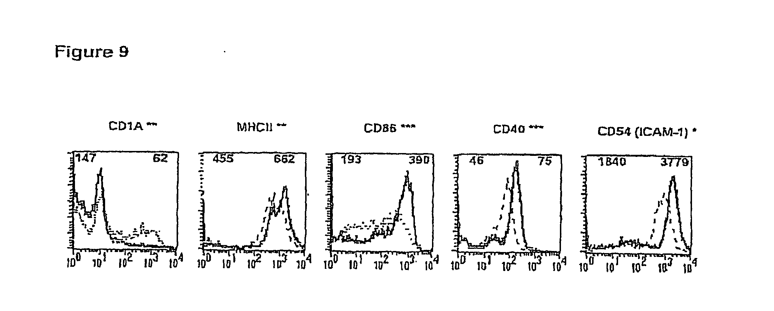

As demonstrated by the histograms of Figure 9, NCM (IRX-2) treatment of immature DCs (indicated by solid lines in the histograms) resulted in the down-regulation of CD1a expression (147 vs. 62) as well as the up-regulation of MHCII expression (455 vs. 662). In addition, NCM treatment led to an increase in cell size and a decrease in granularity (data not shown). Untreated controls are indicated by dashed lines in each histogram. The mean values for untreated DCs are shown in the left upper corner of the panels; the respective values for DCs treated with NCM are shown in the upper right corner. Histograms shown are from a representative experiment and the values represent mean results from at least 10 individual experiments (*=*p*<0.05, **=*p*<0.002, ***=*p*<0.00005, paired Students *t*-test). As further indicated by Figure 9, NCM (IRX-2) treatment enhanced the expression of co-stimulatory surface molecules CD86 (also known as B7-2) (193 vs. 390), CD40 (46 vs. 75), and CD54 (also known as intercellular adhesion molecule 1 or ICAM-1)(1840 vs. 3779). All of these changes in surface marker expression indicate that the NCM of the invention is a potent effector of DC activation.

Consistent with their role as antigen presenting cells, immature DCs have a high endocytic activity and actively take up antigens. Upon maturation, this activity is down-regulated whereupon the DC is engaged in antigen processing and presentation. Under physiological conditions, the down-regulation of APC endocytosis is associated with an increase in peptide/MHC complexes on the surface leading to enhanced stimulation of T cells. To test the influence of NCM (IRX-2) on endocytosis, DCs were incubated with increasing amounts of NCM (IRX-2) and the ability to internalize FITC-dextran was determined. More specifically, adherent PBMCs (monocytes) were treated with GM-CSF and IL-4 (as described above) for four days and then stimulated with TNF-α (at 1 µg/ml) or with increasing concentrations of NCM (IRX-2) up to the equivalent of 1 ng/ml TNF-α. After 18 hrs, the cells were incubated with FITC-Dextran (Sigma, St. Louis, MO), which was added to a final concentration of 1 mg/ml. The cells were cultured for 30 min at 37° C. After incubation, the cells were washed four times with ice-cold PBS and analyzed by flow cytometry as described above.

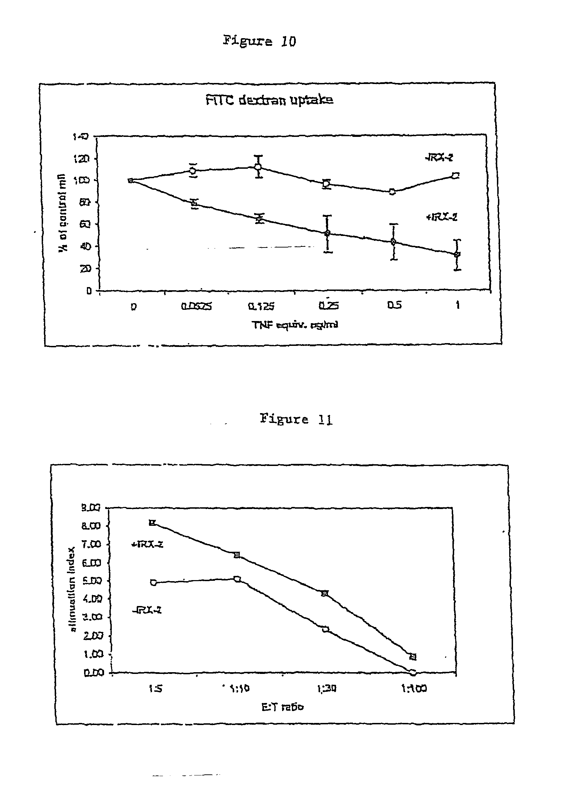

As shown in Figure 10, immature DCs incubated with NCM (IRX-2) (closed circles) down-regulated endocytosis in a dose-dependent manner. TNF-α treatment (open circles) at the corresponding dose found in the NCM had minimal effects. Treatment of immature DCs with higher amounts of TNF-α (10-25 ng/ml) did result in the down-regulation of endocytic activity as expected (data not shown). The data of Figure 10 are shown as the percentage of mean fluorescence intensity of the stimulated versus the unstimulated DCs and are the mean of 4 independent experiments -/+ SEM (p<0.00001, by ANOVA). These experiments indicate that the NCM of the invention down-regulates the endocytic activity of DCs, an indication of DC maturation.

Next, the ability of NCM to enhance the T cell stimulatory capacity of DCs was evaluated. Activated, mature DCs are potent stimulators of naïve T cells. In order to show that NCM (IRX-2) treatment was translated into functional effects as well as the phenotypic and morphologic changes noted above, the influence of NCM (IRX-2) on the T cell stimulatory capacity of DCs was assessed in a mixed lymphocyte reaction (MLR) proliferation assay.

More specifically, adherent PBMCs (monocytes) were first treated with GM-CSF and IL-4 (as described above) for seven days and then stimulated with or without NCM (IRX-2). After 48 hrs, the NCM (IRX-2)-treated or untreated DCs were collected and assayed in an MLR as follows: purified DCs were co-cultured with 1x10⁵ T cells from an unrelated donor at ratios of 1:5, 1:10, 1:30, and 1:100 DC: T cells. Allogeneic T-cells were prepared by running PBMCs purified from buffy coats by FicoII-Hypaque gradient centrifugation over a nylon wool column. The assays were performed in triplicate in round-bottom 96-well plates. No NCM (IRX-2) was present during the MLR assay. After 5 days of DC-T cell co-culture, the wells were pulsed for 18 hours with BrDU. BrDU incorporation was measured using a colorimetric BrDU incorporation assay (Roche Diagnostics, Indianapolis, IN).

As shown in Figure 11, DCs exposed to NCM (IRX-2) (closed squares) two days before co-culture were more potent in inducing a T cell proliferation response than untreated DCs (open circles), confirming that NCM-treated DCs are functionally competent The data in Figure 11 are expressed as stimulation index which is defined as ((o.d. DC stimulated T cell - o.d. DC alone)/o.d. resting T cell) -/+ SEM and are the mean result of 4 individual experiments (*p*<0.05, by ANOVA).

It is important to note that there was no NCM in the co-cultures and the observed increase in T cell stimulation was due to the stimulatory effects of NCM on DCs, rather than a direct effect of the NCM on T cells. Thus, the NCM of the invention enhances the T cell stimulatory activity of DCs as shown by enhanced proliferation in allogenic MLR reactions. Moreover, NCM was shown above to increase the expression of ICAM-1 (CD54). This cell surface accessory ligand has been shown to be involved in signaling through LFA-1 and results in a bias towards a Th1 phenotype (Rogers, 2000). In a cancer setting, the functional consequence of these effects is that NCM-treated DCs would polarize the T cell response towards a Th1 phenotype and favor the activation of tumor specific CTL activity, thus promoting tumor rejection.

Our data also demonstrates that NCM stimulates the production of IL-12 from DCs. IL-12 is a potent Th1 polarizing cytokine secreted by DCs in response to pathogens during infection. However, one of the most important roles of DCs in mediating tumor rejection is to effectively and efficiently stimulate Th1-biased anti-tumor T cell responses and one of the critical cytokines in directing this response is IL-12. IL-12 is produced by activated DCs and is an essential factor involved in the differentiation of naïve CD4⁺ helper T cells into Th1 cells. Th1 cells secrete IFN-γ and IL-2 and these cytokines along with IL-12 mediate the activation and proliferation of cellular and phagocytic components of the immune system, such as CD8⁺ cytotoxic T lymphocytes (CTL).

To determine whether NCM can induce IL-12 production in DCs, GM-CSF/IL₄ cultured monocytes were stimulated with NCM (IRX-2) for 18 hours and assayed for intracellular IL-12 p70 production. More specifically, adherent PBMCs were grown for 4 days in GM-CSF and IL-4 (as described above) and then treated with or without NCM (IRX-2) or LPS for 18 hours. Brefeldin A (BFA; 10 µg/ml; Sigma, St. Louis, MO) was added during the last 4 hours to accumulate most of the cytokine in the Golgi complex. Cells were fixed and permeabilized using Fix and Perm (Caltag, Burlingame, CA), according to the manufacturer's instructions, and were then labeled with FITC-labeled mAb against IL-12 p70 (BD Pharmingen, San Diego, CA) or the appropriate isotype control (BD Pharmingen, San Diego, CA). Cells were analyzed by flow cytometry.

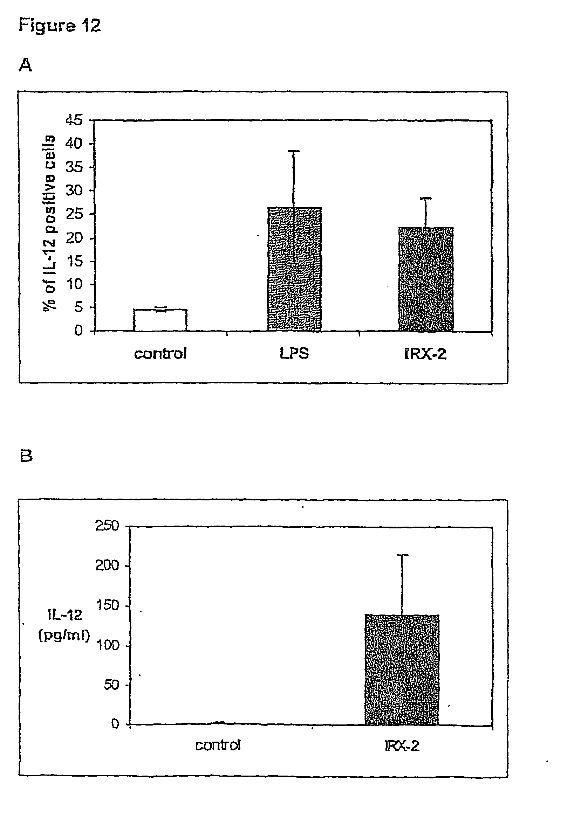

As shown in Figure 12A, NCM (IRX-2) increased the percentage of DCs producing IL-12 from 4.5% positive to 22.5% on average. LPS, a stimulator of IL-12 production in DCs, was used as a positive control and gave similar levels of induction relative to NCM (27% ± 11). The data of Figure 12A is the mean of 4 independent experiments and is expressed as the percentage of cells staining positively for IL-12 -/+ SEM (*p*< 0.05 Students *t-*test). To confirm that the increased intracellular production of IL-12 corresponded to increased secretion of bioactive IL-12, the concentration of bioactive IL-12 in the supernatant of NCM-treated DCs (cultured initially for 4 days with GM-CSF and IL-4 as described above and incubated with NCM for 48 hrs) was measured using a commercial ELISA kit (R&D Systems, Minneapolis, MN) that detects the bioactive p70 heterodimer. Thus, as shown in Figure 12B, 48 hours after exposure to NCM, DC supernatants contained significantly more IL-12 than control-treated DCs. The data of Figure 12B is the mean (-/+ SEM) of 6 independent experiments (*p*<0.05, Students *t*-test).

Finally, our data indicated that NCM reduces VEGF-induced apoptosis in DCs. VEGF is an inhibitor of DC maturation and has been shown to increase apoptosis levels in maturing DCs. To determine if NCM was able to mitigate the effects of VEGF, DCs were treated with VEGF with or without IRX-2 and the level of apoptosis was determined by Annexin-FITC V binding. More specifically, adherent PBMCs were treated with GM-CSF and IL-4 for 7 days and then incubated in the presence or absence of VEGF (100 ng/ml) with or without NCM (IRX-2) (1:3) for 2 additional days. The cells were harvested and washed 2 times in ice-cold PBS and resuspended in Annexin binding buffer (BD Pharmingen, San Diego, CA). Annexin-V FITC (BD Pharmingen, San Diego, CA) and propidium iodide was added and the cells were incubated at 4° C for 30 minutes. Cells were analyzed by flow cytometry.

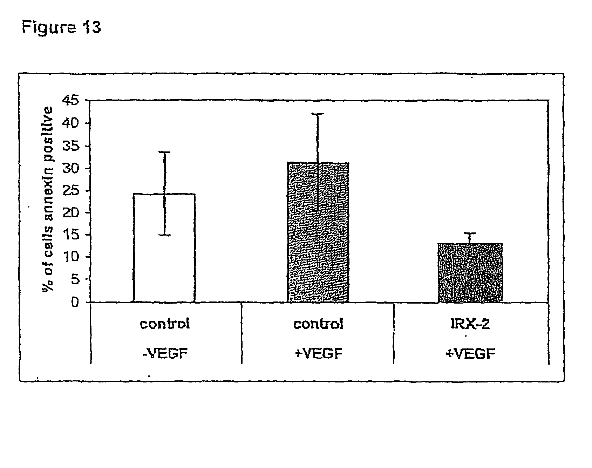

As shown in Figur 13, apoptosis levels increased in VEGF-treated cells as compared to controls; however, NCM (IRX-2) reduced the level of apoptosis in VEGF-treated cells. The data of Figure 13 is the result of 4 independent experiments and is expressed as the percentage of cells staining positively for Annexin V-FITC (-/+ SEM). The data suggests that, in addition to its stimulatory capacity, NCM also has a protective effect on mature DCs. Moreover, defective DC function and number may be mediated in part by aberrant VEGF expression by the tumor (Gabrilovich, 1996b; Saito, 1999; Takahashi, 2004). VEGF production by tumors was shown to be a predictor for poor prognosis in several cancers including H&NSCC, lung cancer, gastric cancer, and osteosarcoma (Gallo, 2001; Kaya, 2000; Miyake, 1992; Saito, 1998; Smith, 2000). The data contained herein indicates that NCM can reverse VEGF-mediated apoptosis of DCs, thus promoting the survival of mature DCs within a tumor environment and allowing for prolonged antigen presentation and activation of tumor antigen-specific cytotoxic T lymphocytes.

Previous studies with DCs have employed natural cytokine mixtures such as monocyte-conditioned media (MCM) or mixtures of recombinant inflammatory cytokines containing TNF-α, IL-1β, IL-6, and PGE₂ to mature DCs for use in *ex vivo* generated DC-based cancer vaccines (Romani, 1996; Bender, 1996; Sorg, 2003). A critical difference between NCM and the cytokine mixtures used in other studies is that the level of cytokines used in this study were 10-100 fold lower, suggesting a significant synergism between the unique cytokine components of NCM. In addition, there are significant problems involved in the use of DCs matured by these other mixtures. For example, DCs matured in the presence of TNF-α, IL-β, IL-6, and PGE₂ have low or absent production of IL-12 and if improperly activated, may be tolerogenic (Steinman, 2002; Langenkamp, 2000). Additionally, there is a concern that fully mature DCs generated *ex vivo* might be "exhausted" and unable to efficiently prime and effective T cell response (Kalinski, 2001). The low levels of clinical responses seen in patients treated with DCs matured by the *ex vivo* method lends support to these concerns (Holtl, 2002; Schuler-Thumer, 2002; Thurner, 1999).

The evidence presented herein confirms that NCM is a potent activator of dendritic cells. This data combined with the known effects of NCM on T cells (Hadden, 1995b) suggests that NCM is able to overcome the APC and T cell defects found in cancer patients and provides a mechanistic explanation for the successful clinical outcomes seen in initial clinical trials. While DCs are now recogrized as central players in cancer-directed immunotherapy, it is becoming increasingly clear that manipulating single elements of the immune system individually, e.g. tumor-specific T cell vaccination strategies or reintroduction of tumor-antigen pulsed DCs alone, is failing to produce significant clinical improvements for patients (Ridgway, 2003; Rosenberg, 2004). A more beneficial treatment plan may be to enhance the activities of several coordinating cell types concomitantly, e.g. T cells and DCs, allowing reinforcing interactions and a better likelihood that functional cascades are perpetuated rather than blocked by the tumor's various immunosuppressive strategies. In this setting, the NCM of the invention may be acting to stimulate both endogenous DCs loaded with tumor antigen and tumor antigen-specific cytotoxic T cells, resulting in an effective immune response and tumor rejection. Taken together these results suggest that NCM is a potentially powerful clinical tool that could be used alone to initiate an immune response against endogenous tumor antigens or could be used in conjunction with exogenously added tumor antigens in a cancer vaccine model.

### Other Uses of the Present Invention for Prognosis:

Historically, there have been few predictors for outcome (positive or negative) in H&NSCC; lymphocyte counts, 1gE and 1gA levels or nutrition were suggested and as mentioned, a DNCB skin test has been used. For chemotherapy (5 FU & cisplatinum), clinical responses occur prior to surgery in the majority of patients, yet mean survival time and overall survival are essentially unaffected. The data presented in the present examples shows that use of the invention delays recurrence of metastasis in those who have residual tumor after surgery and increases survival in a way that relates to the magnitude of the clinical response and the intensity of the immune assault on the tumor as assessed by quantitation of tumor reduction, fragmentation and lymphoid infiltration. These observations point to important modifications of the invention to further improved survival.

### In patients with severe immunodeficiency

In patients with low lymphocyte counts, weak or absent NCM skin tests, sinus histiocytosis, and/or poor pathological responses, retreatment with NCM and monitoring of immune responses would be indicated.

### In Patients with Minor or No Clinical Responses:

These patients have a high risk of recurrence of metastasis and thus would logically benefit from post surgical treatment with the NCM of the present invention. In the absence of currently available tests for tumor rejection response observed in the patients, follow up testing with the triad of tests described in United States Patent No. 6,482,389 would help to determine the frequency of retreatment with the NCM of the present invention.

### In Patients with Recurrent Disease:

Significant responses were observed including two complete responses in patients who were re-treated with the NCM. This is in contrast to previous results with natural and recombinant IL-2, wherein such patients failed to respond to retreatment Thus, the present invention is useful for treating recurrence of disease in patients.

### Use of the Invention with other Treatments like Radiotherapy or Chemotherapy:

Patients with Stage IV H&NSCC cancers have markedly reduced survival compared to patients with Stage III disease (10-20% vs. 30-50%) despite the use of radiotherapy. Radiotherapy is well known to depress T lymphocyte counts in these patients for a prolonged period. Despite the negative impact of radiotherapy on T cell number and function, patients treated with NCM of the present invention having Stage IV disease did as well as patients with Stage III disease. Thus, the therapeutic impact was relatively greater in Stage IV patients, which contradicts current dogma that immunotherapy and cytokine therapy work better with minimal tumor. It also suggests that the NCM of the present invention potentiates the effect of radiotherapy. Similarly, in four patients with penile SCC cancer, the NCM of the present invention was used and was followed by chemotherapy with 5FU and cisplatinum and a second cycle of NCM. Clinical tumor reduction was observed with the initial immunotherapy and chemotherapy. Examination of the tumor (from surgery) showed persistence of immune regression. Another patient with H&N SCC treated with the NCM of the present invention followed by chemotherapy with 5FU and cisplatinum showed the same result. These observations indicate that the NCM of the present invention can be used with chemotherapy.

### Example 4

### Correction of a Monocyte Functional Defect Characterized by a Negative NCM Skin Test

The following results show that NCM containing the six cytokines of IL-1, IL-2, IL-6, IL-8, IFN-γ, and TNF-α is a potent activator of monocytes/macrophages, i.e., when administered by itself (without the administration of CY or INDO).

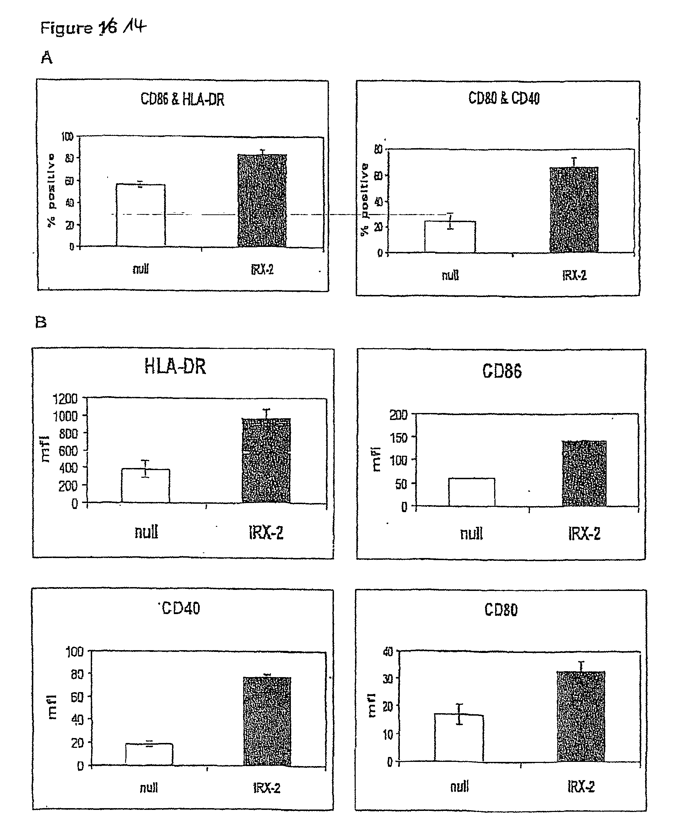

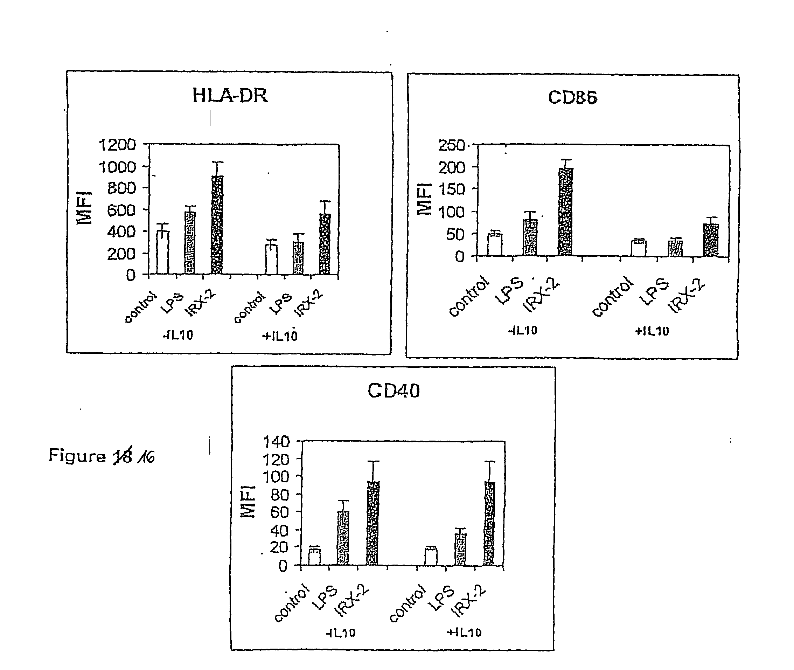

More specifically, adherent PBMCs were grown overnight in X-VIVO 10 media (BioWhittaker Bioproducts), stimulated for 24 hr with NCM (IRX-2) (at a 1:3 final concentration) and assayed for the expression of various activation markers typically found on activated macrophages by flow cytometry. As a control, cells were incubated for 24 hr in media lacking NCM. As demonstrated in Figure 14, the treatment of the cells with NCM versus no added cytokines produced a statistical increase in the percentage of cells staining positively (Figure 14A) and an increase in mean fluorescence index (MFI) (Figure 14B) for HLA-CR, CD86, CD40 and CD80, all activation markers of monocytes/macrophages (p<.03). The data shown in Figure 16 represents the mean value +/- SEM from three independent experiments/donors.

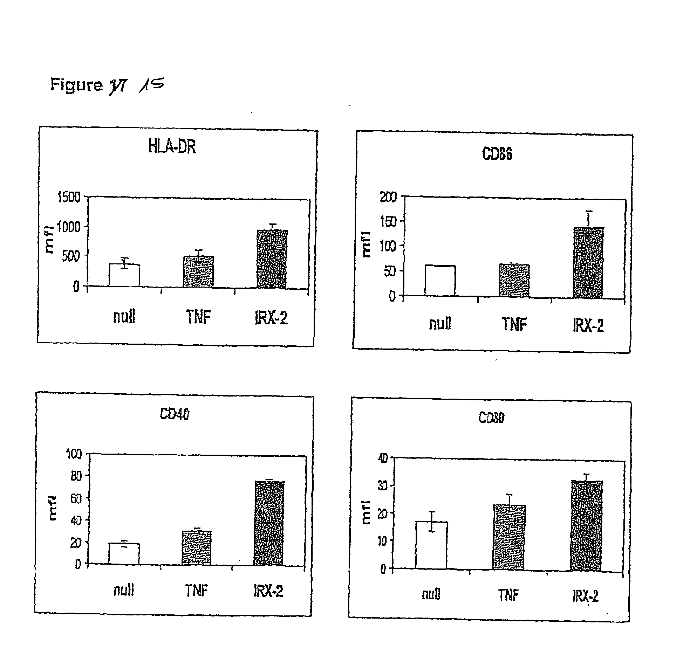

In addition, it was found that the NCM of the invention activates monocytes to a greater degree than TNF-α. More specifically, adherent PBMCs were stimulated with either NCM (IRX-2) (at a 1:3 final concentration; approximately 1 ng/ml TNF-α) or TNF-α (10 ng/ml) and assayed for the expression of activation markers by flow cytometry. As shown in Figure 15, NCM induced statistically greater expression of HLA-DR, CD86, CD40 and CD80 than TNF-α (p<.03). The data shown in Figure 15 represents the mean value +/- SEM from three independent experiments/donors.

Similarly, studies performed using LPS in modest doses (activating but not maximal) also indicated that NCM was a comparatively stronger activation signal. More specifically, adherent PBMCs were stimulated in the absence or presence of IL-10 (5 ng/ml) with either NCM (IRX-2) (at a 1:3 final concentration) or LPS (10 ng/ml) and assayed for the expression of activation markers by flow cytometry. As shown in Figure 16, NCM caused a greater increase in the expression of the monocyte/macrophage maturation markers HLA-DR, CD86, and CD40 than LPS. Moreover, in the presence of the immunosuppressing cytokine, IL-10. the NCM was still able to stimulate the monocytes, whereas LPS failed to do so (p<-02). The data shown in Figure 16 represents the mean value +/- SEM from three independent experiments/donors.

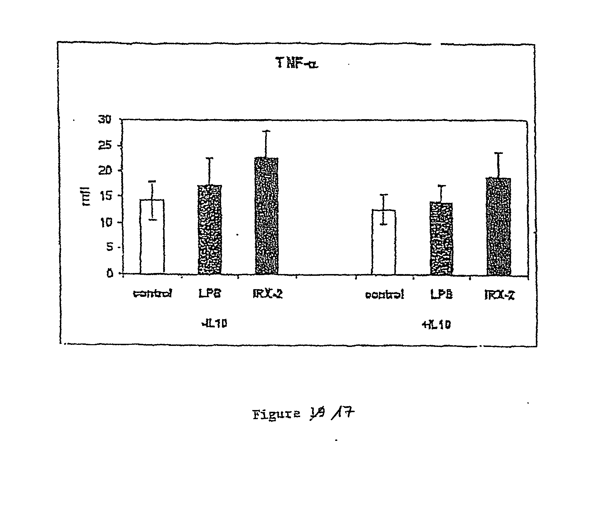

Finally, it is known that monocytes secrete TNF-α in response to activating signals, which secretion is associated with the non-specific killing activity of the monocytes/macrophages. The data shown in Figure 17 demonstrates that the NCM stimulates the production of TNF-α from monocytes and overcomes the immunosuppressive effects of IL-10. More specifically, adherent PBMCs were stimulated in the absence or presence of IL-10 (5 ng/ml) with either NCM (IRX-2) (at a 1:3 final concentration) or LPS (10 ng/ml) and assayed for TNF-α production by intracellular staining and flow cytometry. As shown in Figure 17 NCM caused a greater increase In the production of TNF-α than LPS or controls. In the presence of IL-10, the NCM was still able to stimulate the monocytes to produce TNF-α, whereas LPS was no longer able to do so (p<05). The data shown in Figure 17 represents the mean value +/- SEM from five independent experiments/donors.

The fact that NCM alone has been shown to be a potent activator of monocytes/macrophages supports the contention that NCM treatment alone is responsible for correction of one or more monocyte functional defects characteristic of cancer patients, such as those having a negative NCM skin test

### Publications

Albert et al, Nature, Vol. 392, pp. 86-89 (1998).
Almand B, Resser J, Lindman B, Nadaf S, Clark J, Kwon E, Carbone DP, and Gabrilovich D. Clinical significance of defective dendritic cell differentiation in cancer. Clinical Cancer Research, 6:1755-1766 (2000).
Banchereau et al, Annual Reviews of Immunology, Vol. 18, pp. 767-811 (2000).
Barrera J, Verastegui E, Meneses A, de la Garza J, Zinser J & Hadden JW. Neoadjuvant immunological treatment with IRX-2 in patients with advanced oral cavity squamous cell carcinoma of the head and neck induces clinical and histological responses. In First World Congress on Head and Neck Oncology. JJ Alvarez Vicent, Ed. Monduzzi, Bologna, Italy; 1998; pp 1017-1019.
Barrera J, Verastegui E, Meneses A, Zinser J, de la Garza J, Hadden JW. Combination immunotherapy of squamous cell head and neck cancer. A phase 19 trial. Arch Otolaryngol Head Neck Surg 126:345-351 (2000).
Belldegrun A, Kasid A, Uppenkamp M, Topalian SL, and Rosenberg SA. Human tumor infiltrating lymphocytes: analysis of lymphokine mRNA expression and relevance to cancer immunotherapy. Journal of Immunology, 42:4520-4526 (1989).
Bellone, et al, Immunology Today, Vol 20, No. 10, p 457-462 (1998).
Bender A, Sapp M, Schuler G, Steinamn R, and Bhardwaj N. Improved methods for the generation of dendritic cells from nonproliferating progenitors in human blood. Journal of Immunological Methods, 196:121-135.
Berd D, Maguire Jr HC, Mastrangelo MJ. Potentiation of human cell-mediated and humoral immunity by low-dose cyclophosphamide. Cancer Res 1984; 44:5439-43.
Berd D, Mastrangelo M J. Effect of low dose cyclophosphamide on the immune system of cancer patients: reduction of T suppressor function without depletion of the CD8+subset Cancer Research 47:3317-3321 (1987).
Berd D. Low doses of chemotherapy to inhibit suppressor T cells. Progress in Clin Biol Res 288:449-458 (1989).
Berchtold S, Muhl-Zurbes P, Heufler C, Winklehner P, Schuler G and Steinkasserer. Cloning, recombinant expression and biochemical characterization of the murine CD83 molecule, which is specifically upregulated during dendritic cell maturation. FEBS Letters, 461:211-216 (1999).
Borysiewickz L K, Fiander A. Nimako M. A recombinant vaccine virus encoding human papilomavirus type 16 and 18, E6 and E7 proteins as immunotherapy for cervical cancer. Lancet 347:1524-1527 (1996).
Burke and Olson, "Preparation of Clone Libraries in Yeast Artificial-Chromosome Vectors" in Methods in Enzymology, Vol. 194, "Guide to Yeast 'Genetics and Molecular Biology", eds. C. Guthrie and G. Fink, Academic Press, Inc., Chap. 17, pp. 251-270 (1991).
Capecchi, "Altering the genome by homologous recombination" Science 244:1288-1292 (1989).
Cella M, Scheidegger D, Palmer-Lehamann K, Lane P, Lanzavecchia A, and Alber G. Ligation of CD40 on dendritic cells triggers production of high levels of interleukin-12 and enhances T cell stimulatory capacity: T-T help via APC activation. Journal of Experimental Medicine, 184:747 (1996).
Cella M, Engering A, Pinet V, Pieters J, and Lanzavecchia A. Inflammatory stimuli induce accumulation of MHC II complexes on dendritic cells. Nature, 388:782 (1997).
Chaux P, Moutet M, Faivre J, Martin F, and Martin M. Inflammatory cells infiltrating human colorectal carcinomas express HLA class II but not B7-1 and B7-2 costimulatory molecules of the T-cell activation. Laboratory Investigations, 74:975-983 (1996).
Cortesina G, DeStefani A, Galcazzi E. Temporary regression of recurrent squamous cell carcinoma of the head and neck is achieved with a low dose but not a high dose of recombinant interleukin 2 injected perilymphatically. Br J Cancer 69:572-577 (1994).
Cortesina G, DeStefani A, Giovarelli M, et al. Treatment of recurrent squamous cell carcinoma of the head and neck with low doses of interleukin-2 injected perilymphatically. Cancer 62:2482-2485 (1988).
Cortesina G, Destefani A & Galeazzi E. Temporary regression of recurrent squamous cell carcinoma of the head and neck is achieved with a low but not high dose of recombinant interleukin-2 injected perilymphatically. Br. J. Cancer; 1994; 69: 572-577.
Cowens JW, Ozer H, Ehrke MJ, Colvin M, Mihich E. Inhibition of the development of suppressor cells in culture by 4-hydroperoxycyclophosphamide. J Immunol 1983; 132:95-100.
Cozzolino F, Torcia M, Carossino AM, Giordani R, Selli C, Talini G, Reali E, Novelli A, Pistole V, and Ferrarini M. Characterization of cells from invaded nodes in patients with solid tumors. Lymphokine requirement for tumor specific lymphoproliferative response. Journal of Experimental Medicine, 166:303-318 (1987).
Cregg J M, Vedvick T S, Raschke W C: Recent Advances in the Expression of Foreign Genes in Pichia pastoris, BiolTechnology 11:905-910 (1993).
Cross DS, Platt JL, Juhn SK, et al. Administration of a prostaglandin synthesis inhibitor associated with an increased immune cell infiltrate in squamous cell carcinoma of the head and neck. Arch Otolaryngol Head Neck Surg 1992; 118: 526-8
Culver, Site Directed recombination for repair of mutations in the human ADA gene. (Abstract) Antisense DNA & RNA based therapeutics (1998).
Davies et al., "Targeted alterations in yeast artificial chromosomes for inter-species gene transfer", Nucleic Acids Research, Vol. 20, No. 11, pp. 2693-2698 (1992).
DeLaugh and Lotts, Current Opinion In Immunology, Vol. 12, pp. 583-588 (2000).
DeStefani A, Forni G, Ragona R, et al. Improved survival with perilymphatic interleukin 2 in patients with resectable squamous cell cancer of the inner cavity and oropharynx. Cancer 2002; 95: 90-97
de Vries IJM, Krooshoop DJEB, Scharenborg NM, Lesterhuis WJ, Diepstra JHS, van Muijen GNP, Strijk SP, Ruers TJ, Boerman OC, Oyen WJG, Adema GJ, Punt CJA, and Figdor CG. Effective Migration of Antigen-pulsed Dendritic Cells to Lymph Nodes in Melanoma Patients Is Determined by Their Maturation State. Cancer Research, 63:12-17 (2003).
Dickinson et al., "High frequency gene targeting using insertional vectors", Human Molecular Genetics, Vol. 2, No. 8, pp.1299-1302 (1993).
Duff and Lincoln, "lnsertion of a pathogenic mutation into a yeast artificial chromosome containing the human APP gene and expression in ES cells", Research Advances in Alzheimer's Disease and Related Disorders (1995).
Ehrke MJ, lmmunomodulation in cancer therapies. International Immunopharmacology 2003; 3:1105-19.
Faanes RB, Merluzzi VJ, Ralph P, Williams N, Tarnowski GS. Restoration of tumor and drug-induced immune dysfunction. In: Serrou B, Rosenfeld C, editors. International Symposium on New Trends in Human Immunology and Cancer immunotherapy. Paris: Doin Editeurs; 1980. p. 953-64.
Gabrilovich D, Ciernik F, and Carbone DP. Dendritic cells in anti-tumor immune responses. Defective antigen presentation in tumor-bearing hosts. Cellular Immunology, 170:101-110 (1996a).
Gabrilovich D, Chen H, Girgis K, Cunningham T, Meny G, Nadaf S, Kavanaugh D, and Carbone DP. Production of vascular endothelial growth factor by human tumors inhibits the functional maturation of dendritic cells. Nature Medicine, 2:1096-1103 (1996b).
Gabrilovich D, Corak J, Ciemik IF, Kavanaugh D, and Carbone DP. Decreased antigen presentation by dendritic cells in patients with breast cancer. Clinical Cancer Research, 3:483 (1997).
Gabrilovich D, lshida T, Oyama T, Ran S, Kravtsov V, Nadaf S, and Carbone DP. Vascular endothelial growth factor inhibits the development of dendritic cells and dramatically affects the differentiation of multiple hematopoietic lineages in vivo. Blood, 92:4150-4156 (1998).
Gallo O Franchi A, Magnelli L, Sardi I, Vannacci A, Boddi V, Chiarugi V, and Masini E. Cyclooxygenase-2 Pathway Correlates with VEGF Expression in Head and Neck Cancer. Implications for Tumor Angiogenesis and Metastasis. Neoplasia, 3:53-61 (2001).
Gilboa, E, Eglitis, M A, Kantoff, P W, Anderson, W F: Transfer and expression of cloned genes using retroviral vectors. BioTechniques 4(6):504-512 (1986). Gillis et al. (1978)
Hadden JW, Endicott J, Baekey P, Skipper P, Hadden E M. lnterleukins and contrasuppression induce immune regression of head and neck cancer. Arch Otolaryngol Head Neck Surg. 120:395-403 (1994).
Hadden JW, Saha A R, Sosa M, Hadden E M. Immunotherapy with natural interleukins and/or Thymosin α1 potently augments T lymphocyte responses of hydrocortisone-treated aged mice. Int'I J Immunopharmacol 17:821-828 (1995).
Hadden JW, Verastegui E, Barrera JL, Kurman M, Meneses A, Zinser JW, de la Garza J, and Hadden E. A trial of IRX-2 in patients with squamous cell carcinomas of the head and neck. International Journal of Immunopharmacology, 3:1073-1081 (2003).
Hadden JW, Verastegui E, Barrera J, Meneses A, and de la Garza J. Lymph Node Histology in Head and Neck Cancer Impact of IRX-2. Immunotherapy. Abstract #294 presented at 2004 Annual Meeting of the American Head and Neck Society, Combined Otolaryngology Spring Meetings (COSM), Washington D.C. (2004).
Hadden JW. The Immunology of head and neck cancer prospects for immunotherapy. Clinical Immunotherapy, 3:362-385 (1995a).
Hadden JW. Immunology and immunotherapy of breast cancer: An update: Int'l J Immunopharmacol 21:79-101 (1999).
Hadden JW. The immunopharmacology of head and neck cancer An update. Int'l J Immunopharmacol 11/12:629-644 (1997).
Hadden JW. The treatment of zinc deficiency is an immunotherapy. Int'l J Immunopharmacol 17:696-701 (1995).
Hadden, J., E. Verastegui, J.L. Barrera, M. Kurman, A. Meneses, J.W. Zinser, J. de la Garza, and E. Hadden, "A trial of IRX-2 in patients with squamous cell carcinomas of the head and neck," International lmmunopharmacology 3; 1073-1081 (2003).
Hank A J, Albertini M R, Sondel P M. Monoclonal antibodies, cytokines and fusion proteins in the treatment of malignant disease. Cancer Chemother & Biol Resp Mod 18:210-222 (1999).
Hart DN. Dendritic cells: unique leukocyte population which control the primary immune response. Blood, 90:3245-3287 (1997).
Hengst JCD, Mokyr MB, Dray S. Importance of timing in cyclophosphamide therapy of MOPC-315 tumor bearing mice. Cancer Res 1980; 40:2135-41.
Hengst JCD, Mokyr MB, Dray S. Cooperation between cyclophosphamide tumoricidal activity and host antitumor immunity in the cure of mice bearing large MOPC-315 tumors. Cancer Res 1982; 41:2163-7.
Hirsch B, Johnson JT, Rabin BD, et al. Immunostimulation of patients with head and neck cancer. Arch Otolaryngol 1983; 109: 298-301
Hoffmann T, Muller-Berghaus J, Ferris R, Johnson J, Storkus W, and Whiteside T. Alterations in the Frequency of Dendritic Cell Subsets in the Peripheral Circulation of Patients with Squamous Cell Carcinomas of the Head and Neck. Clinical Cancer Research, 8:1787-1793 (2002).
Holtl L, Zelle-Rieser C, Gander H, Papesh C, Ramoner R, Bartsch G, Rogatsch H, Barsoum AL, Coggin JH Jr., and Thumher M. Immunotherapy of Metastatic Renal Cell Carcinoma with Tumor Lysate-pulsed Autologous Dendritic Cells. Clinical Cancer Research, 8:3369-3376 (2002).
Huston et al, "Protein engineering of single-chain Fv analogs and fusion proteins" in Methods in Enzymology (J J Langone, ed.; Academic Press, New York, N.Y.) 203:46-88 (1991).
Huxley et al., "The human HPRT gene on a yeast artificial chromosome is functional when transferred to mouse cells by cell fusion", Genomics, 9:742-750 (1991).
Jakobovits et al., "Germ-line transmission and expression of a human-derived yeast artificial chromosome", Nature, Vol. 362, pp. 255-261 (1993).
Johnson and Bird, 1991 "Construction of single-chain Fvb derivatives of monoclonal antibodies and their production in Escherichia coli in Methods in Enzymology (J J Langone, ed.; Academic Press, New York, N.Y.) 203:88-99 (1989).
Kavanaugh D Y, Carbone D P. Immunologic dysfunction in cancer. Hematol-Oncol Clinics of North Amer 10(4):927-951 (1996).
Kalinski P, Schuitemaker J, de Jong E, and Kapsenberg M. Prostaglandin E(2) is a selective inducer of interleukin-12 p40 (IL-12p40) production and an inhibitor of bioactive IL-12p70 heterodimer. Blood, 97:3466-3469 (2001).
Kaya M, Wada T, Akatsuka T, Kawaguchi S, Nagoya S, Shindoh M, Higashino F, Mezawa F, Okada F, and Ishii S. Vascular endothelial growth factor expression in untreated osteocarcoma is predictive of pulmonary metastasis and poor prognosis. Clinical Cancer Research, 6:572-578 (2000).
Kleindienst P and Brocker T. Endogenous dendritic cells are required for amplification of T cell responses induced by dendritic cell vaccines in vivo. Journal of Immunology, 170:2817-2823 (2003).
Lamb et al., "Introduction and expression of the 400 kilobase precursor amyloid protein gene in transgenic mice", Nature Genetics, Vol. 5, pp. 22-29 (1993).
Langenkamp A, Messi M, Lanzavecchia A, and Sallusto F. Kinetics of dendritic cell activation: impact on priming of TH1, TH2 and nonpolarized T cells. Nature Immunology, 1:311-316 (2000).
Lissoni P, Matugani F, Bonfanti A, Bucovec R, Secondino S, Brivio F, Ferrari-Bravo A, Ferrante R, Vigoe L, Rovelli F, Mandal M, Viviani S, Fumagalli L, and Gardani G. Abnormally enhanced blood concentrations of vascular endothelial growth factor (VEGF) in metastatic cancer patients and their -relation to circulating dendritic cells, IL-12 and endothelin-1. Journal of Biological Regulatory and Homeostatic Agents, 15:140-144 (2001).
Lou Y, Wang G, Lizee G, Kim GJ, Finkelstein SE, Feng C, Restifo NP, Hwu P. Dendritic cells strongly boost the antitumor activity of adoptively transferred T cells in vivo. Cancer Research, 64:6783-6790 (2004).
Maass G, Schmidt W, Berger M, et al. Priming of tumor-specific T-cells in the draining lymph nodes after immunization with interleukin 2-secreting tumor cells: three consecutive stages may be required for successful tumor vaccination. Proc Natl Acad Sci USA, 92:5540-5542 (1995).
Mackall. Stem Cells 2000, Vol. 18. pp. 10-18.
Mackall, et al. New England Journal of Medicine, Vol. 332, pp. 143-149 (1995).
Maclean G D, Miles D W, Rubens R D, Reddish M A, Longenecker bone marrow. Enhancing the effect of Theratope STn-KLH cancer vaccine in patients with metastatic breast cancer by pretreatment with low-dose intravenous cyclophosphamide. J Immunother Emphasis Tumor Immunol 19(4):309-316 (1996).
Marshak et al, "Strategies for Protein Purification and Characterization. A laboratory course manual." CSHL Press (1996).
Mastrangelo M J, Maguire H C Jr., Sato T, Nathan F E, Berd D. Active specific immunization in the treatment of patients with melanoma. (Review) Seminars in Oncology 23(6):773-781 (1996).
Matzinger P. Tolerance, danger, and the extended family. Annual Review of Immunology, 12:991-1045 (1994).
Meneses A, Verastegui E, Barrera J L, Zinser J, de la Garza J, Hadden J W. Histological findings in patients with head and neck squamous cell carcinoma receiving perilympatic natural cytokine mixture prior to surgery. Arch Pathol Lab Med 122:447-454 (1998).
Meneses A, Verastegui E, Barrera J L, de la Garza J, and Hadden J W, "Lymph node histology in head and neck cancer: impact of immunotherapy with IRX-2," International Immunopharmacology, 3; 1083-1091 (2003).
Mernaugh and Mernaugh, "An overview of phage-displayed recombinant antibodies" in Molecular Methods In Plant Pathology (R P Singh and U S Singh, eds.; CRC Press Inc., Boca Raton, Fla.) pp. 359-365 (1995).
Middel P, Fayyazi A, Kaboth U, and Radzun HJ. Sinus histiocytosis with massive lymphadenopathy: evidence for its relationship to macrophages and for a cytokine-related disorder. Histopathology, 35:525-533 (1999).
Mishell and Shiigi. Selected Methods in Cellular Immunology (1981).
Miyake M, Taki T, Hitomi S, and Hakomori S. Correlation of expression of H/Le(y)/Le(b) antigens with survival in patients with carcinoma of the lung. New England Journal of Medicine, 327:14-19 (1992).
Mokyr MB, Hengst JCD, Dray S. The role of antitumor immunity in cyclophosphamide-induced rejection of subcutaneous non-palpable MOPC-315 tumors. Cancer Res 1982; 42:974-9.
Montovani A, Sozzani S, Locati M, et al. Macrophage polarization: tumor associated macrophages as a paradigm for polarized M2 mononuclear phagocytes. Trends in Immunol 2002; 23: 549-555
Murphy GP, Tjoa B A, Simmons SJ. The prostate. 38:43-78 (1999).
Nestle F, Burg G, Fah J, Wrone-Smith T, and Nickoloff B. Human sunlight-induced basal-cell-carcinoma-associated dendritic cells are deficient in T cell co-stimulatory molecules and are impaired as antigen presenting cells. American Journal of Pathology, 150:641-651 (1997).
Panje WR. Regression of head and neck carcinoma with a prostaglandin-synthesis inhibitor. Arch Otolaryngol 1981; 107: 658-63
Pearson and Choi, Expression of the human b-amyloid precursor protein gene from a yeast artificial chromosome in transgenic mice. Proc. Natl. Acad. Sci. USA, 90:10578-82 (1993).
Randolph G. Dendritic cell migration to lymph nodes: cytokines, chemokines and lipid mediators. Seminars in Immunology, 13:267 (2001).
Ridgway D. The First 1000 Dendritic Cell Vaccines. Cancer Investigation, 21:873-886 (2003).
Roitt I, Brostoff J, Male D. Immunology, JB Lippincott Co, Phila, Pa., (1989).
Romani N, Reider D, Heuer M, Ebner S, Kampgen E, Eibl B, Niederwieser D, and Schuler G. Generation of mature dendritic cells from blood. An improved method with regard to clinical applicability. Journal of Immunological Methods, 196:137-151 (1996).
Rogers P, and Croft M. CD28, Ox-40, LFA-1 and CD-4 modulation of Th1/Th2 differentiation is directly dependent on the dose of the antigen. The Journal of Immunology, 164:2955-2963 (2000).
Rosenberg SA, Yang JC, and Restifo NP. Cancer immunotherapy: moving beyond current vaccines. Natural Medicine, 10:909-915 (2004).
Rothstein, "Targeting, disruption, replacement, and allele rescue: integrative DNA transformation in yeast" in Methods in Enzymology, Vol. 194, "Guide to Yeast Genetics and Molecular Biology", eds. C. Guthrie and G. Fink, Academic Press, Inc., Chap. 19, pp. 281-301 (1991).
Saha A, Hadden E M, Hadden J W. Zinc induces thymulin secretion from human thymic epithelial cells in vitro and augments splenocytes and thymocyte response in vivo. Int'l J lmmunopharmacol 17:729-734 (1995).
Sahin U, Tureci 0, Pfreundschuh. Serological identification of human tumor antigens. Curr Opin Immunol 9:709-715 (1997).
Saito H, Tsujitani S, Ikeguchi M, Maeta M, and Kaibara N. Relationship between the expression of vascular endothelial growth factor and the density of dendritic cells in gastric adenocarcinoma tissue. British Journal of Cancer, 78:1573-1579 (1998).
Saito T, Kuss I, Dworacki G, Gooding W, Johnson J, and Whiteside T. Spontaneous ex Vivi Apoptosis of Peripheral Blood Mononuclear Cells in Patients with Head and Neck Cancer. Clinical Cancer Research, 5:1263-1273 (1999).
Sallusto F, Cella M, Danieli C, and Lanzavecchia A. Dendritic cells use macropinocytosis and the mannose receptor to concentrate macromolecules in the magor histocompatability complex calss II compartment: down regulation by cytokines and bacterial products. Journal of Experimental Medicine, 182:389 (1995).
Sallusto F, and Lanzavecchia A. Efficient presentation of soluble antigen by cultured human dendritic cells is maintained by granulocyte/macrophage colony-stimulating factor plus interleukin 4 and down regulated by tumor necrosis factor α. Journal of Experimental Medicine, 179:1109 (1994).
Sanda M G, Smith D C, Charles L G. Recombinant vaccinia PSA (Prostvac) can include a prostate-specific immune response in androgen-modulated human prostate cancer. Urology 52:2 (1999).
Schedl et al., "A yeast artificial chromosome covering the tyrosinase gene confers copy number-dependent expression in transgenic mice", Nature, Vol. 362, pp. 258-261 (1993).
Schnurr M, Then F, Galambos P, Scholz C, Siegmund B, Endres S, and Eigler A. Extracellular ATP and TNF-α synergize in the activation and maturation of human dendritic cells. Journal of Immunology, 165:4704 (2000).
Schuler-Thumer B, Schultz ES, Berger TG, Weinlich G, Ebner S, Woerl P, Bender A, Feuerstein B, Fritsch PO, Romani N, and Schuler G. Rapid Induction of Tumor-specific Type 1 T Helper Cells in Metastatic Melanoma Patients by Vaccination with Mature, Cryopreserved, Peptide-loaded Monocyte-derived Dendritic Cells. J. Exp. Med., 195:1279-1288 (2002).
Smith B, Smith G, Carter D, Sasaki C, and Haffty B. Prognostic Significance of Vascular Endothelial Growth Factor Protein Levels in Oral and Oropharyngeal Squamous Cell Carcinoma. Journal of Clinical Oncology, 18:2048-2052 (2000).
Sorg R, Ozcan Z, Brefort T, Fischer J, Ackermann R, Muller M, and Wernet P. Clinical-Scale Generation of Dendritic Cells in a Closed System. Journal of Immunotherapy, 26:374-384 (2003).
Sozzani S, Allavena P, D'Amico G, Luini W, Bianchi G, Kataura M, Imai T, Yoshie O, Bonecchi R, and Mantovani A. Differential regulation of chemokine receptors during dendritic cell maturation: a model for their trafficking properties. Journal of Immunology, 161, 1083 (1998).
Sprent, et al, Science, Vol. 293, 245-248 (2001).
Steinman RM. The dendritic cell system and its role in immunogenicity. Annual Review of Immunology, 9:271-296 (1991).
Steinman R, and Nussenzweig M. Avoiding horro autotoxicus: The importance of dendritic cells in peripheral T cell tolerance. Proceedings of the National Academy of Science USA, 99:351-358 (2002).
Strauss et al., "Germ line transmission of a yeast artificial chromosome spanning the murine a1 (I) collagen locus", Science, Vol. 259, pp. 1904-1907 (1993).
Tagawa M. Cytokine therapy for cancer. Current Pharmaceut Design 6(6):681-699 (2000).
Takahashi A, Kono K, Ichihara F, Sugai H, Fujii H, and Matsumoto Y. Vascular Endothelial Growth Factor Inhibits Maturation of Dendritic Cells Induced by Lipopolysaccharide, but not by Proinflammatory Cytokines. Cancer Immunology and Immunotherapy, 53:543-550 (2004).
Tas M, Simons P, Balm F, and Drexhage H. Depressed monocyte polarization and clustering of dendritic cells in patients with head and neck cancer: in vitro restoration of this immunosuppression by thymic hormones. Cancer Immunology and Immunotherapy, 36:108-114 (1993).
Thumher M, Radmayr C, Ramoner R, Ebner S, Bock G, Klocker H, Romani N, and Bartsch G. Human renal-cell carcinoma tissue contains dendritic cells. International Journal of Cancer, 68:1-7 (1996).
Thumher B, Haendle I, Roder C, Dieckmann D, Keikavoussi P, Jonuleit H, Bender A, Maczek C, Schreiner D, von den Driesch P, Brocker EB, Steinman RM, Enk A, Kampgen E, and Schuler G. Vaccination with Mage-3A1 Peptide-pulsed Mature, Monocyte-derived Dendritic Cells Expands Specific Cytotoxic T Cells and Induces Regression of Some Metastases in Advanced Stage IV Melanoma. Journal of Experimental Medicine, 190:1669-1678 (1999).
Valente G, DeStefani A, Jemma C, Giovarelli M, Geuna N, Cortesina G, Fomi G, Palestro G. Infiltrating leukocyte populations and T-lymphocyte subsets in head and neck squamous cell carcinomas from patients receiving perilymphatic injections of recombinant interleukin-2. A pathologic and immunophenotypic study. Modem Pathol 3(6):702-708 (1990).
Valente G, DeStefani A, Jemma C. Infiltrating leukocyte populations and T lymphocyte subsets in H&NSCC from patients receiving perilymphatic injections of rIL-2. Mod Pathol 1990; 3:702-708
Van der Eynde B, Van der Bruggen, T cell defined tumor antigens. Curr Opin Immunol 9:684-693 (1997).
Verastegui E, Barrera J L, Zinzer J, del Rio R, Meneses A, de la Garza J, Hadden J W. A natural cytokine mixture (IRX-2) and interference with immune suppression induce immune mobilization and regression of head and neck cancer. Int'l J Immunopharmacol 11/12:619-627 (1997).
Verastegui E, Hadden EM, Barrera J, Meneses A, Hadden JW. A natural cytokine mixture (IRX-2) and interference with immune suppression induce immune regression and improved survival in head and neck cancer. Int'l. Journal of Immunorehabilitation; 1999; 12: 5-11.
Wang R F, Rosenberg S A. Human tumor antigens for cancer-vaccine development Immunologic Reviews 170:85-100 (1999).
Weber J. Tumor vaccines. Medscape Anthology 3:2 (2000).
Whiteside, et al, Cancer Res. 53:5654-5662, (1993).
Whiteside T, Stanson J, Shurin M, and Ferrone S. Antigen-Processing Machinery in Human Dendritic Cells: Up-Regufation by Maturation and Down--Regulation by Tumor Cells. The Journal of Immunology, 173:1526-1534 (2004).
Whiteside T. Immunobiology and immunotherapy of head and neck cancer. Current Oncology Report, 2001:346-355 (2001).
Wolf et al, Arch. Oto. Laryngol. 111:716-725 (1985).
Zhou L, and Tedder T. CD14+ blood monocytes can differentiate into functionally mature CD83+ dendritic cells. Proceedings of the National Academy of Science USA, 93:2588 (1996).

## Claims

1. A composition comprising a pharmaceutical acceptable carrier and a natural cytokine mixture prepared in the continuous presence of a 4-aminoquinolone antibiotic and with the continuous or pulsed presence of a mitogen, the natural cytokine mixture containing as active components the cytokines IL-1, IL-2, IL-6, IL-8, IFN-γ, and TNF-α, wherein the IL-1 is at a concentration of 60 to 6,000 pcg per ml, the IL-2 is at a concentration of 600 to 60,000 pcg per ml, the IL-6 is at a concentration of 60 to 6,000 pcg per ml, the IL-8 is at a concentration of 6,000 to 600,000 pcg per ml and the IFN-γ and TNF-α are at a concentration of 200 to 20,000 pcg per ml, for use in the treatment of cellular immunodeficiency **characterised by** T lymphocytopenia, by one or more dendritic cell functional defects, or by one or more monocyte functional defects, wherein the treatment comprises administration of said mixture without accompanying administration of cyclophosphamide or indomethacin.

2. A composition for use according to claim 1, wherein the IL-1 is at a concentration of 150 to 1,200 pcg per ml, the IL-2 is at a concentration of 3,000 to 12,000 pcg per ml, the IL-6 is at a concentration of 300 to 2,000 pcg per ml, the IL-8 is at a concentration of 20,000 to 180,000 pcg per ml and the IFN-γ and TNF-α are at a concentration of 1,000 to 4,000 pcg per ml.

3. A composition for use according to claim 1 or 2, in which the mixture further comprises IL-12, GM-CSF, and G-CSF.

4. A composition for use according to any of claims 1 to 3, wherein the cytokine mixture is for administration at a concentration of 40 to 500 units of IL-2 equivalence.

5. A composition for use according to any of claims 1 to 4, wherein the monocyte functional defects have been determined by a negative skin test to said cytokine mixture.

6. A composition for use according to any of claims 1 to 4, wherein the dendritic cell functional defects are associated with lymph node sinus histiocytosis.

7. A composition for use according to any of claims 1 to 6, wherein the treatment is during recurrence of tumours.

## Patentansprüche

1. Zusammensetzung, umfassend einen pharmazeutisch zulässigen Träger und ein natürliches Zytokingemisch, das unter kontinuierlichem Vorhandensein eines 4-Aminochinolon-Antibiotikums und unter kontinuierlichem oder gepulstem Vorhandensein eines Mitogens zubereitet ist, wobei das natürliche Zytokingemisch als aktive Komponenten die Zytokine IL-1, IL-2, IL-6, IL-8, IFN-γ und TNF-α enthält, wobei das IL-1 in einer Konzentration von 60 bis 6.000 pg pro ml, das IL-2 in einer Konzentration von 600 bis 60.000 pg pro ml, das IL-6 in einer Konzentration von 60 bis 6.000 pg pro ml, das IL-8 in einer Konzentration von 6.000 bis 600.000 pg pro ml vorhanden ist und das IFN-γ und das TNF-α in einer Konzentration von 200 bis 20.000 pg pro ml vorhanden sind, für die Verwendung bei der Behandlung von zellulärer Immunschwäche, **gekennzeichnet durch** T-Lymphozytopenie, **durch** mindestens einen funktionellen Defekt dendritischer Zellen oder **durch** mindestens einen funktionellen Defekt von Monozyten, wobei die Behandlung die Verabreichung des Gemisches ohne begleitende Verabreichung von Cyclophosphamid oder Indomethacin umfasst.

2. Zusammensetzung zur Verwendung nach Anspruch 1, wobei das IL-1 in einer Konzentration von 150 bis 1.200 pg pro ml, das IL-2 in einer Konzentration von 3.000 bis 12.000 pg pro ml, das IL-6 in einer Konzentration von 300 bis 2.000 pg pro ml, das IL-8 in einer Konzentration von 20.000 bis 180.000 pg pro ml vorhanden ist und das IFN-γ und das TNF-α in einer Konzentration von 1.000 bis 4.000 pg pro ml vorhanden sind.

3. Zusammensetzung zur Verwendung nach Anspruch 1 oder 2, wobei das Gemisch des Weiteren IL-12, GM-CSF und G-CSF umfasst.

4. Zusammensetzung zur Verwendung nach einem der Ansprüche 1 bis 3, wobei das Zytokingemisch für die Verabreichung in einer Konzentration von 40 bis 500 IL-2 Äquivalenzeinheiten bestimmt ist.

5. Zusammensetzung zur Verwendung nach einem der Ansprüche 1 bis 4, wobei die funktionellen Defekte von Monozyten durch einen negativen Hauttest auf das Zytokingemisch festgestellt wurden.

6. Zusammensetzung zur Verwendung nach einem der Ansprüche 1 bis 4, wobei die funktionellen Defekte dendritischer Zellen mit Lymphknotensinushistiozytose assoziiert sind.

7. Zusammensetzung zur Verwendung nach einem der Ansprüche 1 bis 6, wobei die Behandlung während des Wiederauftretens von Tumoren stattfindet.

## Revendications

1. Composition comprenant un vecteur pharmaceutiquement acceptable et un mélange de cytokines naturelles préparée en présence continue d'un antibiotique 4-aminoquinolone et en présence continue ou pulsée d'un mitogène, le mélange de cytokines naturelles contenant en tant que composants actifs les cytokines IL-1, IL-2, IL-6, IL-8, IFN-γ et TNF-α, dans laquelle l'IL-1 est présente en une concentration de 60 à 6 000 pcg par ml, l'IL-2 est présente en une concentration de 600 à 60 000 pcg par ml, l'IL-6 est présente en une concentration de 60 à 6 000 pcg par ml, l'IL-8 est présente en une concentration de 6 000 à 600 000 pcg par ml et l'IFN-γ et le TNF-α sont présents en une concentration de 200 à 20 000 pcg par ml, destinée à être utilisée dans le traitement de l'immunodéficience cellulaire **caractérisée par** une lymphocytopénie T, par un ou plusieurs défauts fonctionnels des cellules dendritiques, ou par un ou plusieurs défauts fonctionnels des monocytes, dans laquelle le traitement comprend l'administration dudit mélange sans administration concomitante de cyclophosphamide ou d'indométhacine.

2. Composition destinée à être utilisée selon la revendication 1, dans laquelle l'IL-1 est présente en une concentration de 150 à 1 200 pcg par ml, l'IL-2 est présente en une concentration de 3 000 à 12 000 pcg par ml, l'IL-6 est présente en une concentration de 300 à 2 000 pcg par ml, l'IL-8 est présente en une concentration de 20 000 à 180 000 pcg par ml et l'IFN-γ et le TNF-α sont présents en une concentration de 1 000 à 4 000 pcg par ml.

3. Composition destinée à être utilisée selon la revendication 1 ou 2, dans laquelle le mélange comprend en outre l'IL-12, le GM-CSF et le G-CSF.

4. Composition destinée à être utilisée selon l'une quelconque des revendications 1 à 3, dans laquelle le mélange de cytokines est destiné à être administré en une concentration de 40 à 500 unités d'équivalence d'IL-2.

5. Composition destinée à être utilisée selon l'une quelconque des revendications 1 à 4, dans laquelle les défauts fonctionnels des monocytes ont été déterminés par un test cutané négatif au dit mélange de cytokines.

6. Composition destinée à être utilisée selon l'une quelconque des revendications 1 à 4, dans laquelle lesdits défauts fonctionnels des cellules dendritiques sont associés à une histiocytose sinusale des ganglions lymphatiques.

7. Composition destinée à être utilisée selon l'une quelconque des revendications 1 à 6, dans laquelle le traitement a lieu pendant la récurrence tumorale.
